# EUROPEAN PATENT APPLICATION

(11) **EP 1 552 845 A1**
(43) Date of publication of application: **13.07.2005**
(21) Application number: 03761796.6
(22) Date of filing: 25.06.2003
(51) Int. Cl.: A61K 38/17, A61K 39/395, A61K 45/00, A61K 48/00, A61P 35/00, A61P 43/00, C12N 15/09, C07K 16/32, C12Q 1/02, C12Q 1/68

(54) **PREVENTIVES/REMEDIES FOR CANCER**

(30) Priority: 26.06.2002 JP 2002186799; 26.06.2002 JP 2002186815
(71) Applicant: Takeda Pharmaceutical Company Limited, Osaka-shi, Osaka 541-0045 (JP)
(72) Inventor: TANAKA, Hiroshi, Tsukuba-shi, Ibaraki 305-0051 (JP); KAIEDA, Isao, Toride-shi, Ibaraki 302-0023 (JP); HONDA, Kohei, Tsukuba-shi, Ibaraki 305-0821 (JP)
(74) Representative: Rickard, Timothy Mark Adrian
(86) International application number: PCT/JP2003/008036
(87) International publication number: WO 2004/002514

(57) **Abstract**

The invention provides prophylactic/therapeutic agent for cancer, etc. More specifically, a compound or its salt inhibiting the activity f a protein having an amino acid sequence, which is the same or substantially the same amino acid sequence represented by SEQ ID NO: 1 or SEQ ID NO: 16, a compound or its salt inhibiting the expression of a gene for the above protein, an antisense nucleotide containing a base sequence or a part thereof, which is complementary or substantially complementary to the base sequence of a DNA encoding the above protein or its partial peptide, an antibody to the above protein or its partial peptide, etc. are usable as a prophylactic/therapeutic agent for cancer, and so on.

SEQUENCE LISTING

<110> Takeda Chemical Industries, Ltd.

<120> Preventing and treating agent for cancer

<130> P03-0038PCT

<150> JP 2002-186799
<151> 2002-06-26

<150> JP 2002-186815
<151> 2002-06-26

<160> 26

<210> 1
<211> 296
<212> PRT
<213> Human

<400> 1
Met Glu His Leu Lys Ala Phe Asp Asp Glu Ile Asn Ala Phe Leu Asp
                5                   10                  15
Asn Met Phe Gly Pro Arg Asp Ser Arg Val Arg Gly Trp Phe Thr Leu
            20                  25                  30
Asp Ser Tyr Leu Pro Thr Phe Phe Leu Thr Val Met Tyr Leu Leu Ser
        35                  40                  45
Ile Trp Leu Gly Asn Lys Tyr Met Lys Asn Arg Pro Ala Leu Ser Leu
    50                  55                  60
Arg Gly Ile Leu Thr Leu Tyr Asn Leu Gly Ile Thr Leu Leu Ser Ala
65                  70                  75                  80
Tyr Met Leu Ala Glu Leu Ile Leu Ser Thr Trp Glu Gly Gly Tyr Asn
                85                  90                  95
Leu Gln Cys Gln Asp Leu Thr Ser Ala Gly Glu Ala Asp Ile Arg Val
            100                 105                 110
Ala Lys Val Leu Trp Trp Tyr Tyr Phe Ser Lys Ser Val Glu Phe Leu

        115                 120                 125
Asp Thr Ile Phe Phe Val Leu Arg Lys Lys Thr Ser Gln Ile Thr Phe
    130                 135                 140
Leu His Val Tyr His His Ala Ser Met Phe Asn Ile Trp Trp Cys Val
145                 150                 155                 160
Leu Asn Trp Ile Pro Cys Gly Gln Ser Phe Phe Gly Pro Thr Leu Asn
                165                 170                 175
Ser Phe Val His Ile Leu Met Tyr Ser Tyr Tyr Gly Leu Ser Val Phe
            180                 185                 190
Pro Ser Met His Lys Tyr Leu Trp Trp Lys Lys Tyr Leu Thr Gln Ala
        195                 200                 205
Gln Leu Val Gln Phe Val Leu Thr Ile Thr His Thr Met Ser Ala Val
    210                 215                 220
Val Lys Pro Cys Gly Phe Pro Phe Gly Cys Leu Ile Phe Gln Ser Ser
225                 230                 235                 240
Tyr Met Leu Thr Leu Val Ile Leu Phe Leu Asn Phe Tyr Val Gln Thr
                245                 250                 255
Tyr Arg Lys Lys Pro Met Lys Lys Asp Met Gln Glu Pro Pro Ala Gly
            260                 265                 270
Lys Glu Val Lys Asn Gly Phe Ser Lys Ala Tyr Phe Thr Ala Ala Asn
        275                 280                 285
Gly Val Met Asn Lys Lys Ala Gln
    290                 295

<210> 2
<211> 888
<212> DNA
<213> Human

<400> 2
atggaacatc taaaggcctt tgatgatgaa atcaatgctt ttttggacaa tatgtttgga 60
ccgcgagatt ctcgagtcag agggtggttc acgttggact cttaccttcc tacctttttt 120
cttactgtca tgtatctgct ctcaatatgg ctgggtaaca agtatatgaa gaacagacct 180
gctctttctc tcaggggtat cctcaccttg tataatcttg gaatcacact tctctccgcg 240
tacatgctgg cagagctcat tctctccact tgggaaggag gctacaactt acagtgtcaa 300
gatcttacca gcgcagggga agctgacatc cgggtagcca aggtgctttg gtggtactat 360
ttctccaaat cagtagagtt cctggacaca attttcttcg ttttgcggaa aaaaacgagt 420

cagattactt ttcttcatgt atatcatcat gcttctatgt ttaacatctg gtggtgtgtc 480
ttgaactgga taccttgtgg acaaagtttc tttggaccaa cactgaacag ttttgtccac 540
attcttatgt actcctacta tggactttct gtgtttccat ctatgcacaa gtatctttgg 600
tggaagaaat atctcacaca ggctcagctg gtgcagttcg tgctcaccat cacgcacacc 660
atgagcgccg tcgtgaaacc gtgtggcttc cccttcggtt gtctcatctt ccagtcatct 720
tatatgctaa cgttagtcat cctcttctta aatttttatg ttcagacata ccgaaaaaag 780
ccaatgaaga aagatatgca agagccacct gcagggaaag aagtgaagaa tggtttttcc 840
aaagcctact tcactgcagc aaatggagtg atgaacaaga aagcacaa 888

<210> 3
<211> 4002
<212> DNA
<213> Human

<400> 3
gatagcgccg ggcagaggga cccggctacc ctggacagcg catcgccgcc cgcccgggtc 60
gccgcgccac agccgctgcg gatcatggaa catctaaagg cctttgatga tgaaatcaat 120
gcttttttgg acaatatgtt tggaccgcga gattctcgag tcagagggtg gttcacgttg 180
gactcttacc ttcctacctt ttttcttact gtcatgtatc tgctctcaat atggctgggt 240
aacaagtata tgaagaacag acctgctctt tctctcaggg gtatcctcac cttgtataat 300
cttggaatca cacttctctc cgcgtacatg ctggcagagc tcattctctc cacttgggaa 360
ggaggctaca acttacagtg tcaagatctt accagcgcag gggaagctga catccgggta 420
gccaaggtgc tttggtggta ctatttctcc aaatcagtag agttcctgga cacaattttc 480
ttcgttttgc ggaaaaaaac gagtcagatt acttttcttc atgtatatca tcatgcttct 540
atgtttaaca tctggtggtg tgtcttgaac tggatacctt gtggacaaag tttctttgga 600
ccaacactga acagttttgt ccacattctt atgtactcct actatggact ttctgtgttt 660
ccatctatgc acaagtatct ttggtggaag aaatatctca cacaggctca gctggtgcag 720
ttcgtgctca ccatcacgca caccatgagc gccgtcgtga aaccgtgtgg cttccccttc 780
ggttgtctca tcttccagtc atcttatatg ctaacgttag tcatcctctt cttaaatttt 840
tatgttcaga cataccgaaa aaagccaatg aagaaagata tgcaagagcc acctgcaggg 900
aaagaagtga agaatggttt ttccaaagcc tacttcactg cagcaaatgg agtgatgaac 960
aagaaagcac aataaaaatg agtaacagaa aaagcacata tactagccta acagattggc 1020
ttgttttaaa gcaaagactg aattgaaggt tacatgtttt aggataaact aatttctttt 1080
gagttcataa atcatttgta cccagaatgt attaatatat tgctattagg ttaatctgtt 1140
aactgaatgc tttgatcagc attgaggtga tgctcacctc cgaggacctc agaactggtg 1200
cagcttctct ctccctccct cccacagact gaacctttcg ccagaagctg tccttataac 1260
gccttatacg catacacagc caggaaacgt ggagcattgt ttctcacaga gagtctccaa 1320

ataaaaaggg ttttgttcag attaaaatgt ttacaacaaa atgttaatta tattctaaat 1380
acagggtatg ttctaatcta tattaagcaa taatgccagt gcataatcat tccatttgtt 1440
cctttagcaa tcaaccccag aaaatattaa aatgggatca tacacagaag atagaaaaat 1500
ctagcaaaac ttctctttct gtaagccaga gtcttgtcta tcagattccc acaaccactc 1560
ctgattctaa atttagtgat atggtaatga aattggtatt tattttaaat attagttatt 1620
ctaaggagaa aaaaatgctt ctgcaagatt ttcataattc aggggctgtg gataggattg 1680
ttcctctgtt tccctaatca ttcatctgtt catgtctccc tcttgtgcca gtcagcctag 1740
gttatacaga tgccatgctc cacaccacga gcagtgtaca aatctggctg cccgtttact 1800
ttctgagcaa gcactggagt ccactccgac ctttttcttt gaacatgcat gctgctggaa 1860
tatgtataaa tcagaactag cagaagtagc agagtgatgg gagcaaaata ggcactgaat 1920
tcgtcaactc ttttttgtga gcctacttgt gaatattacc tcagatacct gttgtcactc 1980
ttcacaggtt atttaagttt ttgaagctgg gaggaaaaag atggagtagc ttggaaagat 2040
tccagcactg agccgtgggc cggtcatgag ccacgataaa aaatgccagt ttggcaaact 2100
cagcactcct gttccctgct caggtatatg cgatctctac tgagaagcaa gcacaaaagt 2160
agacaaaagt attaatgagt atttcctttc tccataagtg caggactgtt actcactact 2220
aaactctacc aagaatggaa acaaagaata ttttctgaag atttttttga agattaattt 2280
ataccctata aaataaaact tgttagcttc gatgaagtca cttcatcttc tctcctacct 2340
tattttttta aataagtttt taggtcctga cactgacatc aaatacatgc acaccagaaa 2400
ggcatttcca ccaccgtccc cactcattag cgtccagagt gcctttctct ctcggctttt 2460
tttcccccct gagctctagt tttaaacttt ctcctgttaa aaaaattgta cttttatttc 2520
atgtaaactg cccctctgag gatttgggca tattttttgg aaggtgccta atgcttagga 2580
tagtctctag ggtgatgcac tgcacctgct tccttccctt cagtgcggcc gacccatttc 2640
tgttgaacag atgtctcctg tgtgatgccc ctgactctta catttatctt tctaaatcat 2700
tttctgaagc tgacttgctc tagggtgctt catcaccact tccatcttgc atcaccttat 2760
aactgttctg tcttgttctc tcccagcaat ttattttaaa acaagggcta gcttcaagca 2820
tgactgttaa tggctcctct gtgacaaaac acaagttgga ccaaggagaa agccctttgg 2880
agaaactgga ccctagttca gtttagatct caaatatagg ctgaaatctc ttaccaaagt 2940
gcgttccaga taaaaatgag acttagaaga cagactggtc taatatgctc actggtcatc 3000
acattcagct tattcttacg tgccagtgag gccctaaggt ctcagtgcat agcatacctg 3060
ccactctgga tgttttcctg ggcttcatat ctgtatggca aggagggagc tgtccttcag 3120
cagacaaagt caagcagctt taagtacaag gcagcttgaa acctctctga tgaaagtgaa 3180
aaatgtgggt agcttatact ttcaaactaa tgccagtgac tagagagagg tttcattcat 3240
gatgtgtatt tccaatttgg tcttcaattc aagagatgct tcataaaaca cttatagcct 3300
taaaccattc aagggctaat taaaaaaata gttatgaaat cttagccaca aaaaataaat 3360
caggtaaaca ttttaacctg tcgttaagtg ttgtaacttc aaaaaaccaa aatgcttatt 3420
ttaatgtgat ttttctaaaa ctattgaaaa attaatattt ctataatcta ttaaaaaaaa 3480
tggagaatgt tttctctcaa atttcctctg acccatggga atgaaaatac ttaaatactg 3540

taaagcatat ctattaataa ttcccccaat tttttaaact aacaaaatgg aatgttaact 3600
gaatggaatt aaacatagca attgtcaagc catcaaaatt atatatcgaa ccacttaggc 3660
atgttacggg gtaaagtcct cacccctgca tttgagaaat attttattat ttttatttta 3720
aggcaaaaca gtgtttactg tgatgggcag aacagccttt tgtatctagg agtccatttg 3780
agttctttcc aatatttcca tatcgttcat aaatgtgtct gggggcttcc ttgtttggaa 3840
aatatggata tcgctttgtt ttcctttgaa agtaattata tcttaagaac atagtatcat 3900
gaatggagta gacagggagg ctgcagaagc catttctctt tgtagataaa aagcattatc 3960
tatgattgtt gtataataaa ttgattttta cattaaaaaa aa 4002

<210> 4
<211> 39
<212> DNA
<213> Artificial Sequence

<220>
<223> Primer

<400> 4
atgacggagg ttgtgaggca ctgcccccac catgagcgc 39

<210> 5
<211> 39
<212> DNA
<213> Artificial Sequence

<220>
<223> Primer

<400> 5
gcgctcatgg tgggggcagt gcctcacaac ctccgtcat 39

<210> 6
<211> 24
<212> DNA
<213> Artificial Sequence

<220>

<223> Primer

<400> 6
cctgtcgtta agtgttgtaa cttc                    24

<210> 7
<211> 29
<212> DNA
<213> Artificial Sequence

<220>
<223> Primer

<400> 7
attgtttaat tccattcagt taacattcc                    29

<210> 8
<211> 37
<212> DNA
<213> Artificial Sequence

<220>
<223> Primer

<400> 8
ccggaattca tggaacatct aaaggccttt gatgatg                    37

<210> 9
<211> 39
<212> DNA
<213> Artificial Sequence

<220>
<223> Primer

<400> 9
ccggcggccg cttattgtgc tttcttgttc atcactcca                    39

<210> 10
<211> 20
<212> DNA
<213> Artificial Sequence

<220>
<223> Antisense oligonucleotide designed for ELOVL2

<400> 10
agccacacgg tttcacgacg                                    20

<210> 11
<211> 20
<212> DNA
<213> Artificial Sequence

<220>
<223> Primer

<400> 11
gcagcacttt ggcacaccga                                    20

<210> 12
<211> 21
<212> DNA
<213> Artificial Sequence

<220>
<223> Primer

<400> 12
gctcaccatc acgcacacca t                                    21

<210> 13
<211> 20
<212> DNA

<213> Artificial Sequence

<220>
<223> Primer

<400> 13
aaccgaaggg gaagccacac                                    20

<210> 14
<211> 39
<212> DNA
<213> Artificial Sequence

<220>
<223> Primer

<400> 14
gcgaattcag aacatctaaa ggcctttgat gatgaaatc                                    39

<210> 15
<211> 39
<212> DNA
<213> Artificial Sequence

<220>
<223> Primer

<400> 15
cgtctagatt attgtgcttt cttgttcatc actccattt                                    39

<210> 16
<211> 479
<212> PRT
<213> Human

<400> 16
Met Leu Gln Ile Asn Gln Met Phe Ser Val Gln Leu Ser Leu Gly Glu

                5                   10                  15
Gln Thr Trp Glu Ser Glu Gly Ser Ser Ile Lys Lys Ala Gln Gln Ala
            20                  25                  30
Val Ala Asn Lys Ala Leu Thr Glu Ser Thr Leu Pro Lys Pro Val Gln
        35                  40                  45
Lys Pro Pro Lys Ser Asn Val Asn Asn Asn Pro Gly Ser Ile Thr Pro
    50                  55                  60
Thr Val Glu Leu Asn Gly Leu Ala Met Lys Arg Gly Glu Pro Ala Ile
65                  70                  75                  80
Tyr Arg Pro Leu Asp Pro Lys Pro Phe Pro Asn Tyr Arg Ala Asn Tyr
                85                  90                  95
Asn Phe Arg Gly Met Tyr Asn Gln Arg Tyr His Cys Pro Val Pro Lys
            100                 105                 110
Ile Phe Tyr Val Gln Leu Thr Val Gly Asn Asn Glu Phe Phe Gly Glu
        115                 120                 125
Gly Lys Thr Arg Gln Ala Ala Arg His Asn Ala Ala Met Lys Ala Leu
    130                 135                 140
Gln Ala Leu Gln Asn Glu Pro Ile Pro Glu Arg Ser Pro Gln Asn Gly
145                 150                 155                 160
Glu Ser Gly Lys Asp Met Asp Asp Asp Lys Asp Ala Asn Lys Ser Glu
                165                 170                 175
Ile Ser Leu Val Phe Glu Ile Ala Leu Lys Arg Asn Met Pro Val Ser
            180                 185                 190
Phe Glu Val Ile Lys Glu Ser Gly Pro Pro His Met Lys Ser Phe Val
        195                 200                 205
Thr Arg Val Ser Val Gly Glu Phe Ser Ala Glu Gly Glu Gly Asn Ser
    210                 215                 220
Lys Lys Leu Ser Lys Lys Arg Ala Ala Thr Thr Val Leu Gln Glu Leu
225                 230                 235                 240
Lys Lys Leu Pro Pro Leu Pro Val Val Glu Lys Pro Lys Leu Phe Phe
                245                 250                 255
Lys Lys Arg Pro Lys Thr Ile Val Lys Ala Gly Pro Glu Tyr Gly Gln
            260                 265                 270
Gly Met Asn Pro Ile Ser Arg Leu Ala Gln Ile Gln Gln Ala Lys Lys
        275                 280                 285
Glu Lys Glu Pro Asp Tyr Val Leu Leu Ser Glu Arg Gly Met Pro Arg
    290                 295                 300

Arg Arg Glu Phe Val Met Gln Val Lys Val Gly Asn Glu Val Ala Thr
305                 310                 315                 320
Gly Thr Gly Pro Asn Lys Lys Ile Ala Lys Lys Asn Ala Ala Glu Ala
                325                 330                 335
Met Leu Leu Gln Leu Gly Tyr Lys Ala Ser Thr Asn Leu Gln Asp Gln
            340                 345                 350
Leu Glu Lys Thr Gly Glu Asn Lys Gly Trp Ser Gly Pro Lys Pro Gly
        355                 360                 365
Phe Pro Glu Pro Thr Asn Asn Thr Pro Lys Gly Ile Leu His Leu Ser
    370                 375                 380
Pro Asp Val Tyr Gln Glu Met Glu Ala Ser Arg His Lys Val Ile Ser
385                 390                 395                 400
Gly Thr Thr Leu Gly Tyr Leu Ser Pro Lys Asp Met Asn Gln Pro Ser
                405                 410                 415
Ser Ser Phe Phe Ser Ile Ser Pro Thr Ser Asn Ser Ser Ala Thr Ile
            420                 425                 430
Ala Arg Glu Leu Leu Met Asn Gly Thr Ser Ser Thr Ala Glu Ala Ile
        435                 440                 445
Gly Leu Lys Gly Ser Ser Pro Thr Pro Pro Cys Ser Pro Val Gln Pro
    450                 455                 460
Ser Lys Gln Leu Glu Tyr Leu Ala Arg Ile Gln Gly Phe Gln Val
465                 470                 475

<210> 17
<211> 1437
<212> DNA
<213> Human

<400> 17
atgcttcaaa taaatcagat gttctcagtg cagctgagtc ttggtgagca gacatgggaa      60
tccgaaggca gcagtataaa gaaggctcag caggctgttg ccaataaagc tttgactgaa     120
tctacgcttc ccaaaccagt tcagaagcca cccaaaagta atgttaacaa taacccaggc     180
agtataactc caactgtgga actgaatggg cttgctatga aaaggggaga gcctgccatc     240
tacaggccat tagatccaaa gccattccca aattatagag ctaattacaa ctttcggggc     300
atgtacaatc agaggtatca ttgcccagtg cctaagatct tttatgttca gctcactgta     360
ggaaataatg aattttttgg ggaaggaaag actcgacaag ctgctagaca caatgctgca     420
atgaaagccc tccaagcact gcagaatgaa cctattccag aaagatctcc tcagaatggt     480

gaatcaggaa aggatatgga tgatgacaaa gatgcaaata agtctgagat cagcttagtg 540
tttgaaattg ctctgaagcg aaatatgcct gtcagttttg aggttattaa agaaagtgga 600
ccaccacata tgaaaagctt tgttactcga gtgtcagtag gagagttctc tgcagaagga 660
gaaggaaata gcaaaaaact ctccaagaag cgcgctgcga ccaccgtctt acaggagctt 720
aaaaaacttc cacctcttcc tgtggtggaa aagccaaaac tattttttaa aaaacgccct 780
aaaacaatag taaaggccgg accagaatat ggccaaggga tgaaccctat tagccgcctg 840
gcgcaaattc aacaggccaa aaaggaaaag gagccggatt atgttttgct ttcagaaaga 900
ggaatgcctc gacgtcgaga atttgtgatg caggtgaagg taggcaatga agttgctaca 960
ggaacaggac ctaataaaaa gatagccaaa aaaaatgctg cagaagcaat gctgttacaa 1020
cttggttata aagcatccac taatcttcag gatcaacttg agaagacagg ggaaaacaaa 1080
ggatggagtg gtccaaagcc tgggtttcct gaaccaacaa ataatactcc aaaaggaatt 1140
cttcatttgt ctcctgatgt ttatcaagag atggaagcca gccgccacaa agtaatctct 1200
ggcactactc taggctattt gtcacccaaa gatatgaacc aaccttcaag ctctttcttc 1260
agtatatctc ccacatcgaa tagttcagct acaattgcca gggaactcct tatgaatgga 1320
acatcttcta cagctgaagc cataggttta aaaggaagtt ctcctactcc cccttgttct 1380
ccagtacaac cttcaaaaca actggaatat ttagcaagga ttcaaggctt tcaggta 1437

<210> 18
<211> 4058
<212> DNA
<213> Human

<400> 18
   ccaatgttgg agccgtctgc aaagtgtccc cggcaagaag aggctgccta ccacaaggac 60
   tttagcttac tttttaaaga ttgaagaaaa aaaagaagac agaaaaagaa gaactcaaag 120
   atacacaaag taatttgaac caaggctcag aagtttttgg agccgtgagg gatacagcag 180
   tttggtcaat attgtcttaa catgcttcaa ataaatcaga tgttctcagt gcagctgagt 240
   cttggtgagc agacatggga atccgaaggc agcagtataa agaaggctca gcaggctgtt 300
   gccaataaag ctttgactga atctacgctt cccaaaccag ttcagaagcc acccaaaagt 360
   aatgttaaca ataacccagg cagtataact ccaactgtgg aactgaatgg gcttgctatg 420
   aaaaggggag agcctgccat ctacaggcca ttagatccaa agccattccc aaattataga 480
   gctaattaca actttcgggg catgtacaat cagaggtatc attgcccagt gcctaagatc 540
   ttttatgttc agctcactgt aggaaataat gaattttttg gggaaggaaa gactcgacaa 600
   gctgctagac acaatgctgc aatgaaagcc ctccaagcac tgcagaatga acctattcca 660
   gaaagatctc ctcagaatgg tgaatcagga aaggatatgg atgatgacaa agatgcaaat 720
   aagtctgaga tcagcttagt gtttgaaatt gctctgaagc gaaatatgcc tgtcagtttt 780
   gaggttatta aagaaagtgg accaccacat atgaaaagct ttgttactcg agtgtcagta 840
   ggagagttct ctgcagaagg agaaggaaat agcaaaaaac tctccaagaa gcgcgctgcg 900
   accaccgtct tacaggagct taaaaaactt ccacctcttc ctgtggtgga aaagccaaaa 960
   ctatttttta aaaaacgccc taaaacaata gtaaaggccg gaccagaata tggccaaggg 1020
   atgaacccta ttagccgcct ggcgcaaatt caacaggcca aaaaggaaaa ggagccggat 1080
   tatgttttgc tttcagaaag aggaatgcct cgacgtcgag aatttgtgat gcaggtgaag 1140
   gtaggcaatg aagttgctac aggaacagga cctaataaaa agatagccaa aaaaaatgct 1200
   gcagaagcaa tgctgttaca acttggttat aaagcatcca ctaatcttca ggatcaactt 1260
   gagaagacag gggaaaacaa aggatggagt ggtccaaagc ctgggtttcc tgaaccaaca 1320
   aataatactc caaaaggaat tcttcatttg tctcctgatg tttatcaaga gatggaagcc 1380
   agccgccaca aagtaatctc tggcactact ctaggctatt tgtcacccaa agatatgaac 1440
   caaccttcaa gctctttctt cagtatatct cccacatcga atagttcagc tacaattgcc 1500
   agggaactcc ttatgaatgg aacatcttct acagctgaag ccataggttt aaaaggaagt 1560
   tctcctactc ccccttgttc tccagtacaa ccttcaaaac aactggaata tttagcaagg 1620
   attcaaggct ttcaggtatg aattaaaagc aaaaacaaaa aacaaaacaa ttcattagcc 1680
   tcagattctt catctgtata catcacaagg ctcattcttg cctgctagta tggcctacat 1740
   gccacttacg ttttaagtta tttaggaaca caaaggacag acaaaaaagc catatgcaca 1800
   tgcctcattt tctcttattt ttgatctatc tagtaattct tttgctgcct gtctcttctc 1860
   cattttcctt cttctttttt aagcattttt catattcttc actgtcttct atttggtctt 1920
   gattaggtgc atctatctct tcgctctgtc ttccacaaac aaaaattctg ccttcagaca 1980
   tttggtgtta gtatttcaca ctcagttctc ccttttttta cataaggatt gagtttcttt 2040
   ttatgatgat tttaccttta tagcaatttt gaattttgca ttctgttgct agtattgatt 2100
   caggtacacc attaagatac aacattctag aagtctatta ccttaggagt taattaaaca 2160
   tgatatttga agaataatga aatgctttat agttgtttga ggcataacaa tgtgtatttg 2220
   ttttactgga tcatgttttg aactgactag ggagggtagc acctgcctca gatagtacca 2280
   acaattctgt ttcactgggt agtctaaaac tagcttatag tttaacttaa cttgttgtgt 2340
   atgtgaattt agggatggaa actttttttc ccctatttat tctttgtttc cttctgggaa 2400
   aaaaccccac aaaaatcagc actcctttat ggatacattg gagcttttga aagaattgta 2460
   aactctagga aggggaaata tctgtgtctt gatttcttag ttgccttgaa aatcatgtac 2520
   tgaactgtaa ccgctaactt gactggatga actagtttgc ttgtgtgtag agagtgtatt 2580
   gcttcctcag atttcactgt tttcatctcc ttttccatct tagtctttat tccttaagac 2640
   caaaaactgt aatatccttt agaaatgctc tagaagatct gtattgtgta gaatgatcat 2700
   gtatttataa atattttaca agtttagatt ataaaatgaa aaagaaggtc atgtgttttg 2760
   gggggtattt tgcatgttcg gatttttttt tcccttcacc gaacccttct gattctttca 2820
   aactattgcc aggtagttgt tagtgtttct aattggactc ttaatatgaa cttcaagaag 2880
   ctgttaccag ttatcggctc tgtcatctga aattttaacc acttaattta aagttacaat 2940
   tttagaattt gtttttgttg ttttacctta agaaacacaa taaatcactg tttaaaaaag 3000
   atctcaattt atataaagta ctggaaaaaa gctaagtaat ttttagttct atctataatc 3060
   tccgtaggat gaattagaaa taaattgtga tgaaaagaaa ttaactgctt atttatgaat 3120
   ctaatcattt agaaatgtct gagagtaaca ctgcattctt atagaaacaa agcacaaatt 3180
   gcattcaagc tctgaattga ttttttgctt ggagctgttg ttacagtagc tgtaattttg 3240
   ctaccagaat gtcttaattt tttaaatttg tttttatttc taagctcttg gcaatgacaa 3300
   taattataat tttaacatat cttcactgta gtcaacatgt agagtctgct tccctcatta 3360
   ttgcattgct aaggcctttt taaaaagctt atgcttacat aatatactct tttttatgga 3420
   cacgttatat gtttccaaat ctgtgtattg tgatttttac atactaatga atataaacag 3480
   gtgattttaa aatattactg tgcttctttg gttgaatgag ctggtattga tgtaaaatac 3540
   tctgtcattg atggaccact acctgcagct aagcagtgag cagaatctcc gggaaatgac 3600
   ttagtctggc cacatgcata gcccatcttc ataatgtcgc agcagaaggt tctttgtggt 3660
   taaaagtttt aaagcctatt tctttatagg taaccctctc aggtatattt acctggtaga 3720
   aaaacatgta gattgtttct attacttaaa tgttttaatt ggactgtagt ttagaaatta 3780
   caggaccagc ttgttacaga ttatacacta ttctgttact tttatttctg aaacttaaaa 3840
   acaataaatt ctttttctgt gtttctaggt tagaactttt ttattttatt gcacactgaa 3900
   cagatactgt tgcttattga atattgtgta aacccttctt tggctttcct tgcccattgc 3960
   aatttgattt aagcctacta gagccattgt atgtgacagc tatattgtat tacaaagtaa 4020
   aaatatatga gtgtattgaa aaaaaaaaaa aaaaaaaa 4058

<210> 19
<211> 39
<212> DNA
<213> Artificial Sequence

<220>
<223> Primer

<400> 19
atgacggagg ttgtgaggca ctgcccccac catgagcgc 39

<210> 20
<211> 39
<212> DNA
<213> Artificial Sequence

<220>
<223> Primer

<400> 20
gcgctcatgg tgggggcagt gcctcacaac ctccgtcat 39

<210> 21
<211> 22
<212> DNA
<213> Artificial Sequence

<220>
<223> Primer

<400> 21
agagaaagcg gacaataacc ag 22

<210> 22
<211> 20
<212> DNA
<213> Artificial Sequence

<220>
<223> Primer

<400> 22
ccaagggaaa tgctcaaagt 20

<210> 23
<211> 39
<212> DNA
<213> Artificial Sequence

<220>
<223> Primer

<400> 23
ccgatatcat gcttcaaata aatcagatgt tctcagtgc 39

<210> 24

<211> 36
<212> DNA
<213> Artificial Sequence

<220>
<223> Primer

<400> 24
ttgcggccgc tcagacggcc gagtttgatt tcttgc 36

<210> 25
<211> 20
<212> DNA
<213> Artificial Sequence

<220>
<223> Antisense oligonucleotide designed for STAU2

<400> 25
gcaccacgac aaaacaatca 20

<210> 26
<211> 20
<212> DNA
<213> Artificial Sequence

<220>
<223> Primer

<400> 26
actaacaaaa cagcaccacg 20

## Description

### TECHNICAL FIELD

The present invention relates to prophylactic/therapeutic agents and diagnostic agents for cancer, and the like.

### BACKGROND ART

Recent progress of studies has established that cancer is a genetic disease in which plural genetic mutations accumulate in somatic cells and as a result growth of the cells becomes uncontrollable. Among them, p53 is a tumor suppressor gene, which abnormalities are most commonly found in human cancers. It is manifested that p53 possesses a diversity of physiological functions such as G1 arrest, apoptosis induction, check-point function when DNA is damaged, etc. These functions are considered to be exhibited by p53 protein acting as a transcription factor to regulate expression of its target gene. For this reason, when abnormalities take place in p53 (variant p53), these functions are not exerted so that the cells are considered to turn cancerous. On the other hand, when variant p53 is transfected into p53-deficient cancer cells, the cancer cells result in forming colonies even in soft agar, or turn to form tumor in tumor-bearing nude mouse model (Nature Genetics, 4, 42-46, 1993), and so on. By these reports, it is suggested that not only variant p53 would lose the functions of normal p53 (wild type p53) but variant p53 itself would also be actively engaged with malignant alteration. It is reported that this variant p53 induces downstream genes, as wild type p53 does (Oncogene, 12, 1941-1952, 1996). Variant p53 is divided into several types based on differences in mutated positions. There is a report that variant p53-bearing cancer in which arginine (R) at position 175 is substituted by histidine (H) exhibits worst prognosis (Cancer Research, 55, 5217-5221, 1995).

ELOVL2 (Genbank Accession No. XM_166355) has homology to the ELO (elongation of very long chain fatty acids protein) family of S. cerevisae and belongs to the ELOVL family (ELOVL1-5), which is an enzyme participating in elongation of long chain fatty acids. The ELOVL family is an enzyme necessary for the synthesis of long chain (unsaturated) fatty acids of at least 20 carbon atoms or sphingolipids.

Staufen homolog 2 (STAU2) (Genomics, 62, 113-118, 1999) is one of human homologs of Drosophila Staufen, which is a RNA binding protein. It is reported that in rat STAU2 takes part in transfer of mRNA in dendrites of hippocampal neurons (Molecular Biology of The Cell, 9, 2945-2953, 1999). STAU2 belongs to the RNA binding protein family, to which PKR (double-stranded RNA-activated protein kinase), TAR RNA binding protein (TRBP), etc. also belong. It is reported that cells overexpressing TRBP exhibited a transformed phenotype (The EMBO Journal, 16, 611-624, 1997), but nothing is reported on any relationship between STAU2 and cancer.

It has been earnestly desired to develop medicaments targeting molecules specifically expressed in cancer cells to inhibit the growth of cancer cells or induce apoptosis.

### DISCLOSURE OF THE INVENTION

In order to solve the foregoing problems, the present inventors have made extensive investigations and found genes (ELOVL2 gene and STAU2 gene discovered as variant p53 induction genes), expression of which markedly increases in cancer tissues. The inventors have further found that apoptosis is induced by suppressing functions of the genes. Based on the findings, the inventors have made further studies. The present invention has thus come to be accomplished.

That is, the present invention provides the following features and so on.
(1) A prophylactic/therapeutic agent for cancer, comprising a compound or its salt inhibiting the activity of a protein comprising the same or substantially the same amino acid sequence as the amino acid sequence represented by SEQ ID NO: 1, its partial peptide, or a salt thereof;
(2) A prophylactic/therapeutic agent for cancer, comprising a compound or its salt inhibiting the expression of a gene for a protein comprising the same or substantially the same amino acid sequence as the amino acid sequence represented by SEQ ID NO: 1, or its partial peptide;
(3) An antisense polynucleotide containing the entire or part of a base sequence complementary or substantially complementary to a base sequence of a polynucleotide encoding a protein comprising the same or substantially the same amino acid sequence as the amino acid sequence represented by SEQ ID NO: 1, or its partial peptide;
(4) The antisense polynucleotide according to (3), which has the base sequence represented by SEQ ID NO: 10;
(5) A pharmaceutical comprising the antisense polynucleotide according to (3);
(6) The pharmaceutical according to (5), which is a prophylactic/therapeutic agent for cancer;
(7) A diagnostic agent comprising the antisense polynucleotide according to (3);
(8) The diagnostic agent according to (7), which is a diagnostic agent for cancer;
(9) An antibody to a protein comprising the same or substantially the same amino acid sequence as the amino acid sequence represented by SEQ ID NO: 1, its partial peptide, or a salt thereof;
(10) A pharmaceutical comprising the antibody according to (9);
(11) The pharmaceutical according to (10), which is a prophylactic/therapeutic agent for cancer;
(12) A diagnostic agent comprising the antibody according to (9);
(13) The diagnostic agent according to (12), which is a diagnostic for cancer;
(14) A diagnostic agent comprising a polynucleotide encoding a protein comprising the same or substantially the same amino acid sequence as the amino acid sequence represented by SEQ ID NO: 1, or its partial peptide;
(15) The prophylactic/therapeutic agent for cancer according to (1), (2), (6) or (11), wherein the cancer is colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor;
(15a) The prophylactic/therapeutic agent for cancer according to (1), (2), (6) or (11), wherein the cancer is breast cancer or prostate cancer;
(16) The diagnostic agent according to (8), (13) or (14), wherein the cancer is colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor;
(16a) The diagnostic agent according to (8), (13) or (14), wherein the cancer is breast cancer or prostate cancer;
(17) A prophylactic/therapeutic agent for cancer comprising a compound or its salt inhibiting the activity of ELOVL2;
(17a) An apoptosis promoting agent comprising a compound or its salt inhibiting the activity of ELOVL2;
(18) A prophylactic/therapeutic agent for cancer comprising a compound or its salt inhibiting the expression of ELOVL2;
(18a) An apoptosis promoting agent comprising a compound or its salt inhibiting the expression of ELOVL2;
(19) A method of screening a prophylactic/therapeutic agent for cancer, which comprises using a protein comprising the same or substantially the same amino acid sequence as the amino acid sequence represented by SEQ ID NO: 1, its partial peptide, or a salt thereof;
(19a) A method of screening a pharmaceutical compound, which comprises using a compound or its salt inhibiting the activity of a protein comprising the same or substantially the same amino acid sequence as the amino acid sequence represented by SEQ ID NO: 1, its partial peptide, or a salt thereof;
(19b) The screening method according to (19a), wherein the pharmaceutical compound is a compound used for the prevention/treatment of cancer and/or a compound having an effect of preventing/treating cancer;
(20) A kit for screening a prophylactic/therapeutic agent for cancer, comprising a protein comprising the same or substantially the same amino acid sequence as the amino acid sequence represented by SEQ ID NO: 1, its partial peptide, or a salt thereof;
(20a) A kit for screening a pharmaceutical compound, comprising a protein comprising the same or substantially the same amino acid sequence as the amino acid sequence represented by SEQ ID NO: 1, its partial peptide, or a salt thereof;
(20b) The screening kit according to (20a), wherein the pharmaceutical compound is a compound used for the prevention/treatment of cancer and/or a compound having an effect of preventing/treating cancer;
(21) A prophylactic/therapeutic agent for cancer, which is obtainable by using the screening method according to (19) or the screening kit according to (20);
(21a) A pharmaceutical compound or its salt, which is obtainable by using the screening method according to (19a) or the screening kit according to (20a);
(21b) The compound or its salt according to (21a), wherein the pharmaceutical compound is a compound used for the prevention/treatment of cancer and/or a compound having an effect of preventing/treating cancer;
(21c) A prophylactic/therapeutic agent for cancer comprising the compound or its salt according to (21b);
(21d) The prophylactic/therapeutic agent for cancer according to (21c), wherein the cancer is breast cancer or prostate cancer;
(21e) A prophylactic/therapeutic agent for breast cancer or prostate cancer, which is obtainable by using the screening method according to (19) or the screening kit according to (20);
(22) A method of screening a prophylactic/therapeutic agent for cancer, which comprises using a polynucleotide encoding a protein comprising the same or substantially the same amino acid sequence as the amino acid sequence represented by SEQ ID NO: 1, or its partial peptide;
(22a) A method of screening a pharmaceutical compound, which comprises using a polynucleotide encoding a protein comprising the same or substantially the same amino acid sequence as the amino acid sequence represented by SEQ ID NO: 1, or its partial peptide;
(22b) The screening method according to (22a), wherein the pharmaceutical compound is a compound used for the prevention/treatment of cancer and/or a compound having an effect of preventing/treating cancer;
(23) A kit for screening a prophylactic/therapeutic agent for cancer, comprising a polynucleotide encoding a protein comprising the same or substantially the same amino acid sequence as the amino acid sequence represented by SEQ ID NO: 1, or its partial peptide;
(23a) A kit for screening a pharmaceutical compound, comprising a polynucleotide encoding a protein comprising the same or substantially the same amino acid sequence as the amino acid sequence represented by SEQ ID NO: 1, or its partial peptide;
(23b) The screening kit according to (23a), wherein the pharmaceutical compound is a compound used for the prevention/treatment of cancer and/or a compound having an effect of preventing/treating cancer;
(24) A prophylactic/therapeutic agent for cancer, which is obtainable by using the screening method according to (22) or the screening kit according to (23);
(24a) The pharmaceutical compound or its salt, which is obtainable by using the screening method according to (22a) or the screening kit according to (23a);
(24b) The compound or its salt according to (24a), wherein the pharmaceutical compound is a compound used for the prevention/treatment of cancer and/or a compound having an effect of preventing/treating cancer;
(24c) A prophylactic/therapeutic agent for cancer comprising the compound or its salt according to (24b);
(24d) The prophylactic/therapeutic agent according to (24c), wherein the cancer is breast cancer or prostate cancer;
(24e) A prophylactic/therapeutic agent for breast cancer or prostate cancer, which is obtainable by using the screening method according to (22) or the screening kit according to (23);
(25) An apoptosis promoting agent comprising a compound or its salt inhibiting the activity of a protein comprising the same or substantially the same amino acid sequence as the amino acid sequence represented by SEQ ID NO: 1, its partial peptide, or a salt thereof;
(26) An apoptosis promoting agent comprising a compound or its salt inhibiting the expression of a gene for a protein comprising the same or substantially the same amino acid sequence as the amino acid sequence represented by SEQ ID NO: 1, or its partial peptide;
(27) An apoptosis promoting agent comprising the antisense polynucleotide according to (3);
(28) An apoptosis promoting agent comprising the antibody according to (9);
(29) A method of screening an apoptosis promoter, which comprises using a protein comprising the same or substantially the same amino acid sequence as the amino acid sequence represented by SEQ ID NO: 1, its partial peptide, or a salt thereof;
(29a) The screening method according to (19a), wherein the pharmaceutical compound is a compound having an apoptosis promoting action;
(30) A method of screening an apoptosis promoter, which comprises using a polynucleotide encoding a protein comprising the same or substantially the same amino acid sequence as the amino acid sequence represented by SEQ ID NO: 1, or its partial peptide;
(30a) The screening method according to (22a), wherein the pharmaceutical compound is a compound having an apoptosis promoting action;
(31) A method of preventing/treating cancer, which comprises administering to a mammal an effective dose of a compound or its salt inhibiting the activity of a protein comprising the same or substantially the same amino acid sequence as the amino acid sequence represented by SEQ ID NO: 1, its partial peptide, or a salt thereof, or an effective dose of a compound or its salt inhibiting the expression of a gene for the protein;
(32) A method of preventing/treating cancer, which comprises inhibiting the activity of a protein comprising the same or substantially the same amino acid sequence as the amino acid sequence represented by SEQ ID NO: 1, its partial peptide, or a salt thereof, or inhibiting the expression of a gene for the protein;
(33) Use of a compound or its salt inhibiting the activity of a protein comprising the same or substantially the same amino acid sequence as the amino acid sequence represented by SEQ ID NO: 1, its partial peptide, or a salt thereof, or a compound or its salt inhibiting the expression of a gene for the protein, to manufacture a prophylactic/therapeutic agent for cancer;
(34) A prophylactic/therapeutic agent for cancer comprising a compound or its salt inhibiting the activity of a protein comprising the same or substantially the same amino acid sequence as the amino acid sequence represented by SEQ ID NO: 16, its partial peptide, or a salt thereof;
(35) A prophylactic/therapeutic agent for cancer comprising a compound or its salt inhibiting the expression of a gene for a protein comprising the same or substantially the same amino acid sequence as the amino acid sequence represented by SEQ ID NO: 16, or its partial peptide;
(36) An antisense polynucleotide containing the entire or part of a base sequence complementary or substantially complementary to a base sequence of a polynucleotide encoding a protein comprising the same or substantially the same amino acid sequence as the amino acid sequence represented by SEQ ID NO: 16, or its partial peptide;
(37) The antisense polynucleotide according to (36), which has the base sequence represented by SEQ ID NO: 25;
(38) A pharmaceutical comprising the antisense polynucleotide according to (36);
(39) The pharmaceutical according to (38), which is a prophylactic/therapeutic agent for cancer;
(40) A diagnostic agent comprising the antisense polynucleotide according to (36);
(41) The diagnostic agent according to (40), which is a diagnostic for cancer;
(42) An antibody to a protein comprising the same or substantially the same amino acid sequence as the amino acid sequence represented by SEQ ID NO: 16, its partial peptide, or a salt thereof;
(43) A pharmaceutical comprising the antibody according to (42);
(44) The pharmaceutical according to (43), which is a prophylactic/therapeutic agent for cancer;
(45) A diagnostic agent comprising the antibody according to (42);
(46) The diagnostic agent according to (45), which is a diagnostic for cancer;
(47) A diagnostic agent for cancer comprising a polynucleotide encoding a protein comprising the same or substantially the same amino acid sequence as the amino acid sequence represented by SEQ ID NO: 16, or its partial peptide;
(48) The prophylactic/therapeutic agent according to (34), (35), (39) or (44), wherein the cancer is colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor;
(49) The diagnostic agent according to (41), (46) or (47), wherein the cancer is colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor;
(50) A prophylactic/therapeutic agent for cancer comprising a compound or its salt having an action of inhibiting the activity of Staufen homolog 2;
(51) A prophylactic/therapeutic agent for cancer comprising a compound or its salt having an action of inhibiting the expression of Staufen homolog 2;
(52) A method of screening a prophylactic/therapeutic agent for cancer, which comprises using a protein comprising the same or substantially the same amino acid sequence as the amino acid sequence represented by SEQ ID NO: 16, its partial peptide, or a salt thereof;
(52a) A method of screening a pharmaceutical compound, which comprises using a protein comprising the same or substantially the same amino acid sequence as the amino acid sequence represented by SEQ ID NO: 16, its partial peptide, or a salt thereof;
(52b) The screening method according to (52a), wherein the pharmaceutical compound is a compound used for the prevention/treatment of cancer and/or a compound having an effect of preventing/treating cancer;
(53) A kit for screening a prophylactic/therapeutic agent for cancer, comprising a protein comprising the same or substantially the same amino acid sequence as the amino acid sequence represented by SEQ ID NO: 16, its partial peptide, or a salt thereof;
(53a) A kit for screening a pharmaceutical compound, comprising a protein comprising the same or substantially the same amino acid sequence as the amino acid sequence represented by SEQ ID NO: 16, its partial peptide, or a salt thereof;
(53b) The screening kit according to (53a), wherein the pharmaceutical compound is a compound used for the prevention/treatment of cancer and/or a compound having an effect of preventing/treating cancer;
(54) A prophylactic/therapeutic agent for cancer, which is obtainable by using the screening method according to (52) or the screening kit according to (53);
(54a) A pharmaceutical compound, which is obtainable by using the screening method according to (52a) or the screening kit according to (53a);
(54b) The compound according to (54a), wherein the pharmaceutical compound is a compound used for the prevention/treatment of cancer and/or a compound having an effect of preventing/treating cancer;
(54c) A prophylactic/therapeutic agent for cancer comprising the compound or its salt according to (54b);
(55) A method of screening a prophylactic/therapeutic agent for cancer, which comprises using a polynucleotide encoding a protein comprising the same or substantially the same amino acid sequence as the amino acid sequence represented by SEQ ID NO: 16, or its partial peptide;
(55a) A method of screening a pharmaceutical compound, which comprises using a polynucleotide encoding a protein comprising the same or substantially the same amino acid sequence as the amino acid sequence represented by SEQ ID NO: 16, or its partial peptide;
(55b) The screening method according to (55a), wherein the pharmaceutical compound is a compound used for the prevention/treatment of cancer and/or a compound having an effect of preventing/treating cancer;
(56) A kit for screening a prophylactic/therapeutic agent for cancer, comprising a polynucleotide encoding a protein comprising the same or substantially the same amino acid sequence as the amino acid sequence represented by SEQ ID NO: 16, or its partial peptide;
(56a) A kit for screening a pharmaceutical compound, comprising a polynucleotide encoding a protein comprising the same or substantially the same amino acid sequence as the amino acid sequence represented by SEQ ID NO: 16, or its partial peptide;
(56b) The screening kit according to (56a), wherein the pharmaceutical compound is a compound used for the prevention/treatment of cancer and/or a compound having an effect of preventing/treating cancer;
(57) A prophylactic/therapeutic agent for cancer, which is obtainable by using the screening method according to (55) or the screening kit according to (56);
(57a) A pharmaceutical compound or its salt, which is obtainable by using the screening method according to (55a) or the screening kit according to (56a);
(57b) The compound or its salt according to (57a), wherein the pharmaceutical compound is a compound used for the prevention/treatment of cancer and/or a compound having an effect of preventing/treating cancer;
(57c) A prophylactic/therapeutic agent for cancer comprising the compound or its salt according to (57b);
(58) An apoptosis promoting agent comprising a compound or its salt inhibiting the activity of a protein comprising the same or substantially the same amino acid sequence as the amino acid sequence represented by SEQ ID NO: 16, its partial peptide, or a salt thereof;
(59) An apoptosis promoting agent comprising a compound or its salt inhibiting the expression of a gene for a protein comprising the same or substantially the same amino acid sequence as the amino acid sequence represented by SEQ ID NO: 16, or its partial peptide;
(60) An apoptosis promoting agent comprising the antisense polynucleotide according to (36);
(61) An apoptosis promoting agent comprising the antibody according to (42);
(62) A method of screening an apoptosis promoter, which comprises using a protein comprising the same or substantially the same amino acid sequence as the amino acid sequence represented by SEQ ID NO: 16, its partial peptide, or a salt thereof;
(63) A method of screening an apoptosis promoter, which comprises using a polynucleotide encoding a protein comprising the same or substantially the same amino acid sequence as the amino acid sequence represented by SEQ ID NO: 16, or its partial peptide;
(64) A method of preventing/treating cancer, which comprises administering to a mammal an effective dose of a compound or its salt inhibiting the activity of a protein comprising the same or substantially the same amino acid sequence as the amino acid sequence represented by SEQ ID NO: 16, its partial peptide, or a salt thereof, or a compound or its salt inhibiting the expression of a gene for the protein;
(65) A method of preventing/treating cancer, which comprises inhibiting the activity of a protein comprising the same or substantially the same amino acid sequence as the amino acid sequence represented by SEQ ID NO: 16, its partial peptide, or a salt thereof, or inhibiting the expression of a gene for the protein;
(66) Use of a compound or its salt inhibiting the activity of a protein comprising the same or substantially the same amino acid sequence as the amino acid sequence represented by SEQ ID NO: 16, its partial peptide, or a salt thereof, or a compound or its salt inhibiting the expression of a gene for the protein, to manufacture a prophylactic/therapeutic agent for cancer.

### BEST MODE FOR CARRYING OUT THE INVENTION

The protein used in the present invention, which has the same or substantially the same amino acid sequence as the amino acid sequence represented by SEQ ID NO: 1 or SEQ ID NO: 16 (hereinafter the protein of the present invention is sometimes referred to as the protein used in the present invention) may be any protein derived from any cells of human and warm-blooded animals (e.g., guinea pig, rat, mouse, fowl, rabbit, swine, sheep, bovine, monkey, etc.) (such as hepatocytes, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophage, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.); or any tissues where such cells are present, such as brain or any of brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc.; the proteins may also be synthetic proteins.

The amino acid sequence having substantially the same amino acid sequence as that represented by SEQ ID NO: 1 includes amino acid sequences having at least about 50% homology, preferably at least about 60% homology, more preferably at least about 70% homology, much more preferably at least about 80% homology, further much more preferably at least about 90% homology and most preferably at least about 95% homology, to the amino acid sequence shown by SEQ ID NO: 1; and so on.

Homology in the amino acid sequence can be measured under the following conditions (an expectation value = 10; gaps are allowed; matrix = BLOSUM62; filtering = OFF) using a homology scoring algorithm NCBI BLAST (National Center for Biotechnology Information Basic Local Alignment Search Tool).

Preferred examples of the protein containing substantially the same amino acid sequence as the amino acid sequence represented by SEQ ID NO: 1 include proteins having substantially the same amino acid sequence as the amino acid sequence represented by SEQ ID NO: 1 and having an activity of substantially the same nature as that of the protein having the amino acid sequence represented by SEQ ID NO: 1, etc.

As the activity of substantially the same nature, there are, for example, a fatty acid elongation activity, a cell growth promoting activity, and the like. The "substantially the same nature" is used to mean that the nature of these activities is equivalent in terms of quality (e.g., physiologically or pharmacologically). Thus, the fatty acid elongation activity, the cell growth promoting activity, etc. are preferably equivalent (e.g., about 0.01 to 100 times, preferably about 0.1 to 10 times, more preferably 0.5 to 2 times), but differences in degree such as a level of these activities, quantitative factors such as a molecular weight of the protein may be present and allowable.

The fatty acid elongation activity can be determined by publicly known methods, e.g., by the method described in Journal of Cell Biology, 707-717, 2000 or with its modifications. Specifically, (a) the extract from the cells into which an expression vector of the protein of the present invention (e.g., ELOVL2) is transfected, (b) a substrate fatty acid, and (c) labeled malonyl CoA are reacted in an appropriate buffer, the labeled fatty acid is extracted from the reaction solution and the amount of the labeled fatty acid is measured, whereby the degree of the fatty acid elongation activity of the protein of the present invention (e.g., ELOVL2) can be determined.

The cell growth promoting activity can be determined by publicly known methods, e.g., by the colony formation assay or with modifications thereof. Specifically, the cells into which an expression vector of the protein of the present invention (e.g., ELOVL2) is transfected are incubated (e.g., incubated in such a medium that selects only the transfected cells), the formed colony is stained and the area is measured, whereby the degree of the cell growth promoting activity of the protein of the present invention (e.g., ELOVL2) can be determined.

The amino acid sequence having substantially the same amino acid sequence as that represented by SEQ ID NO: 16 includes amino acid sequences having at least about 50% homology, preferably at least about 60% homology, more preferably at least about 70% homology, much more preferably at least about 80% homology, further much more preferably at least about 90% homology and most preferably at least about 95% homology, to the amino acid sequence shown by SEQ ID NO: 16; and so on.

Homology in the amino acid sequence can be measured under the following conditions (an expectation value = 10; gaps are allowed; matrix = BLOSUM62; filtering = OFF) using the homology scoring algorithm NCBI BLAST (National Center for Biotechnology Information Basic Local Alignment Search Tool).

Preferred examples of the protein containing substantially the same amino acid sequence as the amino acid sequence represented by SEQ ID NO: 16 include proteins having substantially the same amino acid sequence as the amino acid sequence represented by SEQ ID NO: 16 and having an activity of substantially the same nature as that of the protein having the amino acid sequence represented by SEQ ID NO: 16, etc.

As the activities of substantially the same nature, there are, for example, a cell growth promoting activity, and the like. The substantially the same is used to mean that the nature of these activities is equivalent in terms of quality (e.g., physiologically or pharmacologically). Thus, the cell growth promoting activity is preferably equivalent (e.g., about 0.01 to 100 times, preferably about 0.1 to 10 times, more preferably 0.5 to 2 times), but differences in degree such as a level of these activities, quantitative factors such as a molecular weight of the protein may be present and allowable.

The cell growth promoting activity can be determined by publicly known methods, e.g., by the colony formation assay or with modifications thereof. Specifically, the cells into which an expression vector of the protein of the present invention (e.g., STAU2) is transfected are cultured (e.g., cultured in such a medium that selects only the transfected cells), the formed colony is stained and the area is measured, whereby the degree of the cell growth promoting activity of the protein of the present invention (e.g., STAU2) can be determined.

Examples of the protein used in the present invention include so-called muteins such as proteins having (1) the amino acid sequence represented by SEQ ID NO: 1 or SEQ ID NO: 16, of which at least 1 or 2 (e.g., about 1 to about 100, preferably about 1 to about 30, more preferably about 1 to about 10 and most preferably several (1 to 5)) amino acids are deleted; (2) the amino acid sequence represented by SEQ ID NO: 1 or SEQ ID NO: 16, to which at least 1 or 2 (e.g., about 1 to about 100, preferably about 1 to about 30, more preferably about 1 to about 10 and most preferably several (1 to 5)) amino acids are added; (3) the amino acid sequence represented by SEQ ID NO: 1 or SEQ ID NO: 16, in which at least 1 or 2 (e.g., about 1 to about 100, preferably about 1 to about 30, more preferably about 1 to about 10 and most preferably several (1 to 5)) amino acids are inserted; (4) the amino acid sequence represented by SEQ ID NO: 1 or SEQ ID NO: 16, in which at least 1 or 2 (e.g., about 1 to about 100, preferably about 1 to about 30, more preferably about 1 to about 10 and most preferably several (1 to 5)) amino acids are substituted by other amino acids; or (5) a combination of these amino acid sequences; and the like.

Where the amino acid sequence is inserted, deleted or substituted as described above, the position of its insertion, deletion or substitution is not particularly limited.

Throughout the specification, the proteins are represented in accordance with the conventional way of describing proteins, that is, the N-terminus (amino terminus) at the left hand and the C-terminus (carboxyl terminus) at the right hand. In the protein used in the present invention including the protein having the amino acid sequence represented by SEQ ID NO: 1 or SEQ ID NO: 16, the C-terminus may be in any form of a carboxyl group (-COOH), a carboxylate (-COO-), an amide (-CONH₂) and an ester (-COOR).

Herein, examples of the ester group shown by R include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl such as a phenyl-C₁₋₂ alkyl group, e.g., benzyl, phenethyl, etc.; an α-naphthyl-C₁₋₂ alkyl group such as α-naphthylmethyl, etc.; pivaloyloxymethyl and the like.

Where the protein used in the present invention contains a carboxyl group (or a carboxylate) at a position other than the C-terminus, the carboxyl group may be amidated or esterified and such an amide or ester is also included within the protein used in the present invention. Examples of the ester group in this case may be the C-terminal esters described above, etc.

Furthermore, examples of the protein used in the present invention include variants wherein the amino group at the N-terminal amino acid residues (e.g., methionine residue) is protected with a protecting group (e.g., a C₁₋₆ acyl group such as a C₁₋₆ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a C₁₋₆ acyl group such as a C₁₋₆ alkanoyl group, e.g., formyl group, acetyl group, etc.), or conjugated proteins such as glycoproteins having sugar chains; etc.

Specific examples of the protein used in the present invention are a protein comprising the amino acid sequence represented by SEQ ID NO: 1, a protein comprising the amino acid sequence represented by SEQ ID NO: 16 and the like.

The partial peptide used in the protein of the present invention may be any peptide as long as it is a partial peptide of the protein used in the present invention described above and preferably has the property equivalent to that of the protein used in the present invention described above.

Preferably used are peptides containing, e.g., at least 20, preferably at least 50, more preferably at least 70, much more preferably at least 100, and most preferably at least 200, amino acids in the constituent amino acid sequence of the protein used in the present invention, and the like.

The partial peptide used in the present invention may contain deletion of at least 1 or 2 (preferably about 1 to about 10 and more preferably several (1 to 5)) amino acids in the amino acid sequence; addition of at least 1 or 2 (preferably about 1 to about 20, more preferably about 1 to about 10 and most preferably several (1 to 5)) amino acids in the amino acid sequence; insertion of at least 1 or 2 (preferably about 1 to about 20, more preferably about 1 to about 10 and most preferably several (1 to 5)) amino acids in the amino acid sequence; or substitution of at least 1 or 2 (preferably about 1 to about 10, more preferably several and most preferably about 1 to about 5) amino acids in the amino acid sequence by other amino acids.

In the partial peptide used in the present invention, the C-terminus may be in any form of a carboxyl group (-COOH), a carboxylate (-COO-), an amide (-CONH₂) or an ester (-COOR).

Furthermore, the partial peptide used in the present invention includes variants having a carboxyl group (or a carboxylate) at a position other than the C-terminus, those having an amino group protected with a protecting group at the N-terminal amino acid residues (e.g., methionine residue); those being cleaved at the N-terminal region in vivo and with the glutamyl group thus formed being pyroglutaminated; those having a substituent on the side chain of an amino acid in the molecule wherein the substituent is protected with a suitable protecting group, or conjugated peptides such as so-called glycopeptides having sugar chains; etc., as in the protein used in the present invention described above.

The partial peptide used in the present invention may also be used as an antigen for producing antibodies.

As salts of the protein or partial peptide used in the present invention, salts with physiologically acceptable acids (e.g., inorganic acids or organic acids) or bases (e.g., alkali metal salts) may be employed, preferably in the form of physiologically acceptable acid addition salts. Examples of such salts include salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein or partial peptide used in the present invention or salts thereof may be manufactured by publicly known methods used to purify a protein from human or warm-blooded animal cells or tissues described above. Alternatively, they may also be manufactured by culturing transformants containing DNAs encoding these proteins. Furthermore, they may also be manufactured by a modification of the methods for peptide synthesis, which will be later described.

Where these proteins are manufactured from human or mammalian tissues or cells, human or non-human mammalian tissues or cells are homogenized, extracted with an acid or the like, and the extract is isolated and purified by a combination of chromatography techniques such as reverse phase chromatography, ion exchange chromatography, and the like.

To synthesize the protein or partial peptide used in the present invention or its salts, or amides thereof, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids, in which α-amino groups and functional groups on the side chains are appropriately protected, are condensed on the resin in accordance with the sequence of the objective protein according to various condensation methods publicly known in the art. At the end of the reaction, the protein or partial peptide is excised from the resin and at the same time, the protecting groups are removed. Then, intramolecular disulfide bond-forming reaction is performed in a highly diluted solution to obtain the objective protein or partial peptide, or amides thereof.

For condensation of the protected amino acids described above, a variety of activation reagents for protein synthesis may be used, and carbodiimides are particularly employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be appropriately chosen from solvents that are known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to avoid any possible effect on the subsequent reaction.

Examples of the protecting groups used to protect the starting amino groups include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (linear, branched or cyclic alkyl esterification of, e.g., methyl, ethyl, propyl, butyl, t-butyl, cyclopentyl, cyclohexyl, cycloheptyl, cyclooctyl, 2-adamantyl, etc.), aralkyl esterification (e.g., benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower (C₁₋₆) alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, Cl₂-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting material include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As the amino acids in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid, trifluoroacetic acid, or a mixture solution of these acids; a treatment with a base such as diisopropylethylamine, triethylamine, piperidine or piperazine; reduction with sodium in liquid ammonia, etc. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the desired protein or partial peptide, for example, the α-carboxyl group of the carboxy terminal amino acid is first protected by amidation; the peptide (protein) chain is then extended from the amino group side to a desired length. Thereafter, a protein or partial peptide, in which only the protecting group of the N-terminal α-amino group of the peptide chain has been eliminated, and a protein or partial peptide, in which only the protecting group of the C-terminal carboxyl group has been eliminated are manufactured. The two proteins or peptides are condensed in a mixture of the solvents described above. The details of the condensation reaction are the same as described above. After the protected protein or peptide obtained by the condensation is purified, all the protecting groups are eliminated by the method described above to give the desired crude protein or peptide. This crude protein or peptide is purified by various known purification means. Lyophilization of the major fraction gives the amide of the desired protein or peptide.

To prepare the esterified protein or peptide, for example, the α-carboxyl group of the carboxy terminal amino acid is condensed with a desired alcohol to prepare the amino acid ester, which is followed by procedures similar to the preparation of the amidated protein or peptide above to give the desired esterified protein or peptide.

The partial peptide used in the present invention or salts thereof can be manufactured by publicly known methods for peptide synthesis, or by cleaving the protein used in the present invention with an appropriate peptidase. For the methods for peptide synthesis, for example, either solid phase synthesis or liquid phase synthesis may be used. That is, the partial peptide or amino acids that can construct the partial peptide used in the present invention are condensed with the remaining part. Where the product contains protecting groups, these protecting groups are removed to give the desired peptide. Publicly known methods for condensation and elimination of the protecting groups are described in (1) to (5) below.
(1) M. Bodanszky & M.A. Ondetti: Peptide Synthesis, Interscience Publishers, New York (1966)
(2) Schroeder & Luebke: The Peptide, Academic Press, New York (1965)
(3) Nobuo Izumiya, et al.: Peptide Gosei-no-Kiso to Jikken (Basics and experiments of peptide synthesis), published by Maruzen Co. (1975)
(4) Haruaki Yajima & Shunpei Sakakibara: Seikagaku Jikken Koza (Biochemical Experiment) 1, Tanpakushitsu no Kagaku (Chemistry of Proteins) IV, 205 (1977)
(5) Haruaki Yajima ed.: Zoku Iyakuhin no Kaihatsu (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide used in the present invention. When the partial peptide obtained by the above methods is in a free form, the partial peptide can be converted into an appropriate salt by a publicly known method or its modification; when the partial peptide is obtained in a salt form, it can be converted into a free form or other different salt form by a publicly known method or its modification.

The polynucleotide encoding the protein used in the present invention may be any polynucleotide so long as it contains the base sequence encoding the protein used in the present invention described above. Preferably, the polynucleotide is a DNA. The DNA may also be any one of genomic DNA, genomic DNA library, cDNA derived from the cells or tissues described above, cDNA library derived from the cells or tissues described above and synthetic DNA.

The vector used for the library may be any of bacteriophage, plasmid, cosmid, phagemid and the like. In addition, the DNA can be amplified by reverse transcriptase polymerase chain reaction (hereinafter abbreviated as RT-PCR) with total RNA or mRNA fraction prepared from the above-described cells or tissues.

The DNA encoding the protein used in the present invention may be any one of, for example, (1) a DNA having the base sequence represented by SEQ ID NO: 2, or a DNA comprising a base sequence hybridizable to the base sequence represented by SEQ ID NO: 2 under high stringent conditions and encoding a protein which has the properties of substantially the same nature as those of the protein having the amino acid sequence represented by SEQ ID NO: 1 described above, and (2) a DNA comprising the base sequence represented by SEQ ID NO: 17, or a DNA comprising a base sequence hybridizable to the base sequence represented by SEQ ID NO: 17 under high stringent conditions and encoding a protein which has the properties of substantially the same nature as those of the protein comprising the amino acid sequence represented by SEQ ID NO: 16 described above.

Specific examples of the DNA that is hybridizable to the base sequence represented by SEQ ID NO: 2 under high stringent conditions include DNAs comprising at least about 50% homology, preferably at least about 60% homology, more preferably at least about 70% homology, much more preferably at least about 80% homology, further much more preferably at least about 90% homology and most preferably at least about 95% homology, to the base sequence represented by SEQ ID NO: 2; and the like.

Specific examples of the DNA that is hybridizable to the base sequence represented by SEQ ID NO: 17 under high stringent conditions include DNAs comprising at least about 50% homology, preferably at least about 60% homology, more preferably at least about 70% homology, much more preferably at least about 80% homology, further much more preferably at least about 90% homology and most preferably at least about 95% homology, to the base sequence represented by SEQ ID NO: 17; and the like.

Homology in the base sequence can be measured under the following conditions (an expectation value = 10; gaps are allowed; filtering = ON; match score = 1; mismatch score = -3) using the homology scoring algorithm NCBI BLAST (National Center for Biotechnology Information Basic Local Alignment Search Tool).

The hybridization can be carried out by publicly known methods or by modifications thereof, for example, by the method described in Molecular Cloning, 2nd ed. (J. Sambrook et al., Cold Spring Harbor Lab. Press, 1989). A commercially available library can also be used according to the instructions of the attached manufacturer's protocol. The hybridization can be carried out preferably under high stringent conditions.

The high stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration at about 19 mM at a temperature of about 65°C are most preferred.

More specifically, (1) as the DNA encoding the protein comprising the amino acid sequence represented by SEQ ID NO: 1, there may be employed a DNA comprising the base sequence represented by SEQ ID NO: 2, a DNA comprising the base sequence represented by SEQ ID NO: 3, etc. and (2) as the DNA encoding the protein comprising the amino acid sequence represented by SEQ ID NO: 16, there may be employed a DNA comprising the base sequence represented by SEQ ID NO: 17, a DNA comprising the base sequence represented by SEQ ID NO: 18, etc.

The polynucleotide (e.g., DNA) encoding the partial peptide used in the present invention may be any polynucleotide so long as it contains the base sequence encoding the partial peptide used in the present invention described above. The polynucleotide may also be any of genomic DNA, genomic DNA library, cDNA derived from the cells and tissues described above, cDNA library derived from the cells and tissues described above and synthetic DNA.

As the DNA encoding the partial peptide used in the present invention, there are employed, for example, a DNA comprising a part of the DNA having the base sequence represented by SEQ ID NO: 2 or SEQ ID NO: 17, or a DNA comprising a base sequence hybridizable to the base sequence represented by SEQ ID NO: 2 or SEQ ID NO: 17 under high stringent conditions and comprising a part of DNA encoding a protein having the activities of substantially the same nature as those of the protein of the present invention, and the like.

The DNA hybridizable to the base sequence represented by SEQ ID NO: 2 or SEQ ID NO: 17 indicates the same meaning as described above.

Methods for the hybridization and the high stringent conditions that can be used are the same as those described above.

For cloning of DNAs that completely encode the protein or partial peptide used in the present invention (hereinafter sometimes merely referred to as the protein of the present invention in the description of cloning of DNAs encoding the protein and partial peptide and their expression), the DNA can be either amplified by PCR using synthetic DNA primers containing a part of the base sequence of the protein of the present invention, or the DNA inserted into an appropriate vector can be selected by hybridization with a labeled DNA fragment or synthetic DNA that encodes a part or entire region of the protein of the present invention. The hybridization can be carried out, for example, according to the method described in Molecular Cloning, 2nd (J. Sambrook et al., Cold Spring Harbor Lab. Press, 1989). Where the hybridization is carried out using commercially available library, the procedures may be conducted in accordance with the protocol described in the attached instructions.

Substitution of the base sequence of DNA can be effected by publicly known methods such as the ODA-LA PCR method, the Gapped duplex method, the Kunkel method, etc., or its modification, using PCR, a publicly known kit available as Mutan™-super Express Km (manufactured by Takara Shuzo Co., Ltd.) or Mutan™-K (manufactured by Takara Shuzo Co., Ltd.), etc.

The cloned DNA encoding the protein can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector for the protein of the present invention can be manufactured, for example, by (a) excising the desired DNA fragment from the DNA encoding the protein of the present invention, and then (b) ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pAl-11, pXT1, pRc/CMV, pRc/RSV, pcDNA I/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, it is preferred to use CMV (cytomegalovirus) promoter, SRα promoter, etc. Where the host is bacteria of the genus Escherichia, preferred examples of the promoter include trp promoter, lac promoter, recA promoter, λP_{L} promoter, lpp promoter, T7 promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin prompter, P 10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo^{r}, G418 resistance), etc. In particular, when dhfr gene is used as the selection marker using dhfr gene-deficient Chinese hamster cells, selection can also be made on a thymidine free medium.

If necessary, a signal sequence that matches with a host is added to the N-terminus of the protein of the present invention. Examples of the signal sequence that can be used are PhoA signal sequence, OmpA signal sequence, etc. when bacteria of the genus Escherichia is used as the host; α-amylase signal sequence, subtilisin signal sequence, etc. when bacteria of the genus Bacillus is used as the host; MFα signal sequence, SUC2 signal sequence, etc. when yeast is used as the host; and insulin signal sequence, α-interferon signal sequence, antibody molecule signal sequence, etc. when animal cells are used as the host, respectively.

Using the vector containing the DNA encoding the protein of the present invention thus constructed, transformants can be manufactured.

Examples of the host, which may be employed, are bacteria belonging to the genus Escherichia, bacteria belonging to the genus Bacillus, yeast, insect cells, insects, animal cells, etc.

Specific examples of the bacteria belonging to the genus Escherichia include Escherichia coli K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cereviseae AH22, AH22R⁻, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cell (Sf cell), MG1 cell derived from mid-intestine of Trichoplusia ni, High Five™ cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell which can be used are Sf9 cell (ATCC CRL1711), Sf21 cell (both cells are described in Vaughn, J. L. et al., In Vivo, 13, 213-217 (1977)), etc.

As the insect, for example, a larva of Bombyx mori can be used [Maeda et al., Nature, 315, 592 (1985)].

Examples of animal cells include monkey cell COS-7, Vero, Chinese hamster cell CHO (hereinafter referred to as CHO cell), dhfr gene-deficient Chinese hamster cell CHO (hereinafter simply referred to as CHO (dhfr⁻) cell), mouse L cell, mouse AtT-20, mouse myeloma cell, mouse ATDC5 cell, rat GH3, human FL cell, etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, 52, 456 (1973).

Thus, the transformants transformed with the expression vectors containing the DNAs encoding the protein can be obtained.

Where the host is bacteria belonging to the genus Escherichia or the genus Bacillus, the transformant can be appropriately cultured in a liquid medium which contains materials required for growth of the transformant such as carbon sources, nitrogen sources, inorganic materials, and the like. Examples of the carbon sources include glucose, dextrin, soluble starch, sucrose, etc.; examples of the nitrogen sources include inorganic or organic materials such as ammonium salts, nitrate salts, corn steep liquor, peptone, casein, meat extract, soybean cake, potato extract, etc.; and, examples of the inorganic materials are calcium chloride, sodium dihydrogenphosphate, magnesium chloride, etc. In addition, yeast extracts, vitamins, growth promoting factors etc. may also be added to the medium. Preferably, pH of the medium is adjusted to about 5 to about 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus Escherichia are used as the host, the transformant is usually cultivated at about 15 to 43°C for about 3 to 24 hours. If necessary, the culture may be aerated or agitated.

Where the bacteria belonging to the genus Bacillus are used as the host, the transformant is cultured generally at about 30 to 40°C for about 6 to 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to about 5 to 8. In general, the transformant is cultivated at about 20 to 35°C for about 24 to 72 hours. If necessary, the culture can be aerated or agitated.

Where insect cells or insects are used as the host, the transformant is cultivated in, for example, Grace's Insect Medium (Grace, T. C. C., Nature), 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary, the culture can be aerated or agitated.

Where animal cells are employed as the host, the transformant is cultured in, for example, MEM medium containing about 5 to 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 to 60 hours and, if necessary, the culture can be aerated or agitated.

As described above, the protein of the present invention can be produced in the transformant, in the cell membrane of the transformant, or outside of the transformant.

The protein of the present invention can be separated and purified from the culture described above by the following procedures.

When the protein of the present invention is extracted from the bacteria or cells, the bacteria or cell is collected after culturing by a publicly known method and suspended in an appropriate buffer. The bacteria or cell is then disrupted by publicly known methods such as ultrasonication, a treatment with lysozyme and/or freeze-thaw cycling, followed by centrifugation, filtration, etc to produce crude extract of the protein. Thus, the crude extract of the protein can be obtained. The buffer used for the procedures may contain a protein modifier such as urea or guanidine hydrochloride, or a surfactant such as Triton X-100™, etc. When the protein of the present invention is secreted in the culture broth, the supernatant can be separated, after completion of the cultivation, from the bacteria or cell to collect the supernatant by a publicly known method.

The protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein thus obtained is in a free form, the protein can be converted into the salt by publicly known methods or modifications thereof. On the other hand, when the protein is obtained in the form of a salt, it can be converted into the free form or in the form of a different salt by publicly known methods or modifications thereof.

The protein produced by the recombinant can be treated, prior to or after the purification, with an appropriate protein-modifying enzyme so that the protein can be subjected to addition of an appropriate modification or removal of a partial polypeptide. Examples of the protein-modifying enzyme include trypsin, chymotrypsin, arginyl endopeptidase, protein kinase, glycosidase and the like.

The presence of the thus produced protein of the present invention can be determined by an enzyme immunoassay or western blotting using a specific antibody.

The antibodies to the protein or partial peptide used in the present invention, or its salts may be any of polyclonal and monoclonal antibodies, as long as they are capable of recognizing the protein or partial peptide used in the present invention, or its salts.

The antibodies to the protein or partial peptide used in the present invention, or its salts, (hereinafter they are sometimes collectively referred to as the protein of the present invention in the description of the antibodies) can be produced by a publicly known method of producing an antibody or antiserum, using the protein of the present invention as an antigen.

### [Preparation of monoclonal antibody]

### (a) Preparation of monoclonal antibody-producing cells

The protein of the present invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every about 2 to about 6 weeks and about 2 to about 10 times in total. Examples of the applicable warm-blooded animals are monkeys, rabbits, dogs, guinea pigs, mice, rats, sheep, goats and fowl, with the use of mice and rats being preferred.

In the preparation of monoclonal antibody-producing cells, a warm-blooded animal, e.g., mice, immunized with an antigen wherein the antibody titer is noted is selected, then spleen or lymph node is collected after 2 to 5 days from the final immunization and antibody-producing cells contained therein are fused with myeloma cells from homozoic or heterozoic animal to give monoclonal antibody-producing hybridomas. Measurement of the antibody titer in antisera may be carried out, for example, by reacting a labeled protein, which will be described later, with the antiserum followed by assaying the binding activity of the labeling agent bound to the antibody. The fusion may be carried out, for example, by the known method by Koehler and Milstein [Nature, 256, 495, (1975)]. Examples of the fusion accelerator are polyethylene glycol (PEG), Sendai virus, etc., of which PEG is preferably employed.

Examples of the myeloma cells are those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubation at 20 to 40°C, preferably at 30 to 37°C for 1 to 10 minutes, an efficient cell fusion can be carried out.

Various methods can be used for screening of monoclonal antibody-producing hybridomas. Examples of such methods include a method which comprises adding the supernatant of a hybridoma to a solid phase (e.g., a microplate) adsorbed with the protein as an antigen directly or together with a carrier, adding an anti-immunoglobulin antibody (where mouse cells are used for the cell fusion, anti-mouse immunoglobulin antibody is used) labeled with a radioactive substance or an enzyme or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance or an enzyme and detecting the monoclonal antibody bound to the solid phase, or the like.

The monoclonal antibody can be screened according to publicly known methods or their modifications. In general, the screening can be performed in a medium for animal cells supplemented with HAT (hypoxanthine, aminopterin and thymidine). Any screening and growth medium can be employed as far as the hybridoma can grow there. For example, RPMI 1640 medium containing 1 to 20%, preferably 10 to 20% fetal bovine serum, GIT medium (Wako Pure Chemical Industries, Ltd.) containing 1 to 10% fetal bovine serum, a serum free medium for cultivation of a hybridoma (SFM-101, Nissui Seiyaku Co., Ltd.) and the like, can be used for the screening and growth medium. The culture is carried out generally at 20 to 40°C, preferably at 37°C, for about 5 days to about 3 weeks, preferably 1 to 2 weeks, normally in 5% CO₂. The antibody titer of the culture supernatant of a hybridoma can be determined as in the assay for the antibody titer in antisera described above.

### (b) Purification of monoclonal antibody

Separation and purification of a monoclonal antibody can be carried out by publicly known methods, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody.]

### [Preparation of polyclonal antibody]

The polyclonal antibody of the present invention can be manufactured by publicly known methods or modifications thereof. For example, a warm-blooded animal is immunized with an immunogen (protein antigen) per se, or a complex of immunogen and a carrier protein is formed and a warm-blooded animal is immunized with the complex in a manner similar to the method described above for the manufacture of monoclonal antibodies. The product containing the antibody to the protein of the present invention is collected from the immunized animal followed by separation and purification of the antibody.

In the complex of immunogen and carrier protein used to immunize a warm-blooded animal, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin or hemocyanin is coupled to hapten in a carrier-to-hapten weight ratio of approximately 0.1 to 20, preferably about 1 to 5.

A variety of condensation agents can be used for the coupling of carrier to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 2 to 6 weeks and about 3 to 10 times in total.

The polyclonal antibody can be collected from the blood, ascites, etc., preferably from the blood of warm-blooded animal immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as in the separation and purification of monoclonal antibodies described hereinabove.

The antisense polynucleotide having a complementary or substantially complementary base sequence to the base sequence of a polynucleotide encoding the protein or partial peptide used in the present invention (e.g., DNA (hereinafter these DNAs are sometimes collectively referred to as the DNA of the present invention in the description of antisense polynucleotide)) can be any antisense polynucleotide, so long as it possesses a base sequence complementary or substantially complementary to the base sequence of the polynucleotide (e.g., DNA) of the present invention and capable of suppressing the expression of said DNA, but antisense DNA is preferred.

The base sequence substantially complementary to the DNA of the present invention may include, for example, a base sequence having at least about 70% homology, preferably at least about 80% homology, more preferably at least about 90% homology and most preferably at least about 95% homology, to the entire base sequence or to its partial base sequence (i.e., complementary strand to the DNA of the present invention), and the like. Especially in the entire base sequence of the complementary strand to the DNA of the present invention, preferred are (a) an antisense polynucleotide having at least about 70% homology, preferably at least about 80% homology, more preferably at least about 90% homology and most preferably at least about 95% homology, to the complementary strand of the base sequence which encodes the N-terminal region of the protein of the present invention (e.g., the base sequence around the initiation codon) in the case of antisense polynucleotide directed to translation inhibition and (b) an antisense polynucleotide having at least about 70% homology, preferably at least about 80% homology, more preferably at least about 90% homology and most preferably at least about 95% homology, to the complementary strand of the entire base sequence of the DNA of the present invention having intron, in the case of antisense polynucleotide directed to RNA degradation by RNaseH, respectively.

Specific examples include an antisense polynucleotide containing the entire or part of a base sequence complementary or substantially complementary to a base sequence of DNA containing the base sequence represented by SEQ ID NO: 2 or SEQ ID NO: 17, preferably an antisense polynucleotide containing the entire or part of a base sequence complementary to a base sequence of DNA containing the base sequence represented by SEQ ID NO: 2 or SEQ ID NO: 17 (more preferably, an antisense polynucleotide containing the entire or part of a base sequence complementary to a base sequence of DNA containing the base sequence represented by SEQ ID NO: 2 or SEQ ID NO: 17), etc.

The antisense polynucleotide is generally constituted by bases of about 10 to about 40, preferably about 15 to about 30.

To prevent digestion with a hydrolase such as nuclease, etc., the phosphoric acid residue (phosphate) of each nucleotide that constitutes the antisense DNA may be substituted with chemically modified phosphoric acid residues, e.g., phosphorothioate, methyl phosphonate, phosphorodithionate, etc. Also, the sugar (deoxyribose) in each nucleotide may be replaced by a chemically modified structure such as 2'-O-methylation, etc. The base part (pyrimidine, purine) may also be chemically modified and may be any one which hybridizes to a DNA containing the base sequence represented by SEQ ID NO: 2 or SEQ ID NO: 17. These antisense polynucleotides may be synthesized using a publicly known DNA synthesizer, etc.

According to the present invention, the antisense polynucleotide (nucleic acid) capable of inhibiting the replication or expression of a gene for the protein of the present invention can be designed and synthesized based on the base sequence information of cloned or identified protein-encoding DNA. Such a polynucleotide (nucleic acid) is hybridizable to RNA of a gene for the protein of the present invention to inhibit the synthesis or function of said RNA or is capable of modulating and/or controlling the expression of a gene for the protein of the present invention via interaction with RNA associated with the protein of the present invention. Polynucleotides complementary to the selected sequences of RNA associated with the protein of the present invention and polynucleotides specifically hybridizable to RNA associated with the protein of the present invention are useful in modulating and/or controlling the in vivo and in vitro expression of the protein gene of the present invention, and are useful for the treatment or diagnosis of diseases, etc. The term "corresponding" is used to mean homologous to or complementary to a particular sequence of the nucleotide including the gene, base sequence or nucleic acid. The term "corresponding" between nucleotides, base sequences or nucleic acids and peptides (proteins) usually refer to amino acids of a peptide (protein) under the order derived from the sequence of nucleotides (nucleic acids) or their complements. In the protein genes, the 5' end hairpin loop, 5' end 6-base-pair repeats, 5' end untranslated region, polypeptide translation initiation codon, protein coding region, ORF translation termination codon, 3' end untranslated region, 3' end palindrome region, and 3' end hairpin loop, may be selected as preferred target regions, though any other region may be selected as a target in the protein genes.

The relationship between the targeted nucleic acids and the polynucleotides complementary to at least a part of the target region, specifically the relationship between the target nucleic acids and the polynucleotides hybridizable to the target region, can be denoted to be "antisense." Examples of the antisense polynucleotides include polynucleotides containing 2-deoxy-D-ribose, polynucleotides containing D-ribose, any other type of polynucleotides which are N-glycosides of a purine or pyrimidine base, or other polymers containing non-nucleotide backbones (e.g., commercially available protein nucleic acids and synthetic sequence-specific nucleic acid polymers) or other polymers containing nonstandard linkages (provided that the polymers contain nucleotides having such a configuration that allows base pairing or base stacking, as is found in DNA or RNA), etc. The antisense polynucleotides may be double-stranded DNA, single-stranded DNA, double-stranded RNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., α anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide (nucleic acid) of the present invention is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense nucleic acids of the present invention can be modified preferably based on the following design, that is, by increasing the intracellular stability of the antisense nucleic acid, enhancing the cell permeability of the antisense nucleic acid, increasing the affinity of the nucleic acid to the targeted sense strand to a higher level, or minimizing the toxicity, if any, of the antisense nucleic acid.

Many of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993; etc.

The antisense nucleic acid of the present invention may contain altered or modified sugars, bases or linkages. The antisense nucleic acid may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory action of the antisense nucleic acid can be examined using the transformant of the present invention, the gene expression system of the present invention in vivo and in vitro, or the translation system for the protein of the present invention in vivo and in vitro. The nucleic acid can be applied to cells by a variety of publicly known methods.

Hereinafter, the protein of the present invention, its partial peptides, or salts thereof (hereinafter sometimes merely referred to as the protein of the present invention), the DNA encoding the protein of the present invention or its partial peptides (hereinafter sometimes merely referred to as the DNA of the present invention), the antibodies to the protein of the present invention, its partial peptides, or salts thereof (hereinafter sometimes referred to as the antibodies of the present invention) and the antisense polynucleotides to the DNA of the present invention (hereinafter sometimes merely referred to as the antisense polynucleotides of the present invention) are specifically described for their applications.

Also, the protein or its partial peptide having the same or substantially the same amino acid sequence as the amino acid sequence represented by SEQ ID NO: 1 is sometimes referred to as the protein A of the present invention, and the protein or its partial peptide having the same or substantially the same amino acid sequence as the amino acid sequence represented by SEQ ID NO: 16 is sometimes referred to as the protein B of the present invention.

The protein of the present invention is increasingly expressed in cancer tissues and is thus available as a disease marker. That is, the protein is useful as a marker for early diagnosis in cancer tissues, for judgment of severity in conditions, or for predicted development of these diseases.

Moreover, the antisense polynucleotide of the present invention has an excellent apoptosis promoting action on cancer tissues to promote cell death.

Accordingly, pharmaceuticals containing the antisense polynucleotide of the present invention, the compound or its salt inhibiting the activity of the protein of the present invention, the compound or its salt inhibiting the expression of a gene for the protein of the present invention, or the antibody of the present invention can be used as a prophylactic/therapeutic agent for cancer, e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor, etc.; apoptosis promoters, etc.

Also, the gene for the protein of the present invention is poorly expressed in normal tissues, which enables to screen a prophylactic/therapeutic agent for cancer with fewer side effects. It is further known that cancers harboring variant p53 genes show a high grade of malignancy, showing clinically less effective antitumor drugs, significantly poorer prognosis, etc. The gene for the protein of the present invention acts downstream variant p53 so that the antisense polynucleotide to a polynucleotide encoding the protein of the present invention, the compound or its salts inhibiting the activity of the protein of the present invention, the compound or its salts inhibiting the expression of the gene for the protein of the present invention, the antibody to the protein of the present invention, etc. are effective for highly malignant cancers as well.

### (1) Screening of drug candidate compounds for disease

The protein of the present invention shows increased expression in cancer tissues. In addition, when the activity of the protein of the present invention is inhibited, cancer cells induce apoptosis. Thus, the compound or its salts inhibiting the activity of the protein of the present invention can be used as a prophylactic/therapeutic agent for cancer, including colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor, etc. Preferably, the compound or its salts can be used as a prophylactic/therapeutic agent for breast cancer, prostate cancer, etc. Moreover, the compound or its salts inhibiting the activity of the protein of the present invention can be used as, e.g., apoptosis promoters.

The protein of the present invention is useful as a reagent for screening the compound or its salts inhibiting the activity of the protein of the present invention.

The present invention provides a method of screening the compound or its salts inhibiting the activity (e.g., a fatty acid elongation activity, a cell growth promoting activity, etc.) of the protein A of the present invention, which comprises using the protein A of the present invention.

Specifically, there is employed the method of screening the compound or its salts inhibiting the activity, which comprises comparing (ia) the fatty acid elongation activity or the cell growth promoting activity of a cell capable of producing the protein A of the present invention with (iia) the fatty acid elongation activity or the cell growth promoting activity of a mixture of a cell capable of producing the protein A of the present invention and a test compound.

In the screening method described above, for example, in the cases (ia) and (iia), the fatty acid elongation activity is assayed by publicly known methods, e.g., by the method described in The Journal of Cell Biology, 707-717, 2000, or with its modifications, followed by comparison.

Specifically, the method comprises assaying and comparing the labeled amounts of liberated fatty acids in (ia) the case in which (1) the extract of cells transfected with an expression vector of the protein A (e.g., ELOVL2) of the present invention, (2) a substrate fatty acid and (3) labeled malonyl CoA are reacted in an adequate buffer and the labeled fatty acid is extracted from the reaction solution, and in (iia) the case in which (1) the extract of cells transfected with an expression vector of the protein A (e.g., ELOVL2) of the present invention, (2) a substrate fatty acid, (3) labeled malonyl CoA and (4) a test compound are reacted in an adequate buffer and the labeled fatty acid is extracted from the reaction solution.

Examples of the substrate fatty acids used include unsaturated fatty acids (e.g., eicosapentaenoic acid, docosapentaenoic acid, eicosatetraenoic acid, arachidonic acid, arachidonyl-coenzyme, docosatetraenoic acid, eicosatrienoic acid, mead acid, eicosatetraenoic acid, eicosatrienoic acid, di-homo-gamma-linolenic acid, tetracosateteraenoic acid, tetracosapentaenoic acid, docosapentaenoic acid, octadecenoic acid, eicosenic acid, dococenoic acid, tetracocenoic acid, octadecadienoic acid, eicosadienoic acid, etc.). Preferably unsaturated fatty acids having 18 to 24 carbon atoms (preferably 20 or 22 carbon atoms), etc. are used.
More preferably, cis-5,8,11,14,17-eicosapentaenoic acid (EPA), cis-7,10,13,16,19-docosapentaenoic acid (DPA), cis-5,8,11,14-eicosatetraenoic acid (arachidonic acid), arachinonyl-CoA, cis-7,10,13,16-docosatetraenoic acid, cis-5,8,11-eicosatrienoic acid, etc. are used.

Examples of labeling agents used are radioactive isotopes (e.g., [³H], [¹⁴C], etc.), fluorescent substances [cyanine fluorescent dyes (e.g., Cy2, Cy3, Cy5, Cy5.5, Cy7 (manufactured by Amersham Pharmacia Biotech), etc.), fluorescamine, fluorescein isothiocyanate, etc.] or the like.

The labeled amount may be determined by publicly known methods.

A specific example of the screening method described above is given below.

A fatty acid elongation activity reaction solution [50 mM potassium phosphate/sodium hydroxide (pH 6.5), 5 µM of Rotenone (Wako Pure Chemical Industries, Ltd.), 100 µM of CoA (Wako Pure Chemical Industries, Ltd.), 1 mM ATP, 1 mM NADPH and 1 mM magnesium chloride] containing a substrate fatty acid is prepared. The reaction solution is dispensed in a 96-well plate, followed by incubation at 37°C for 1 minute. Thereafter, a test compound and the extract of cells derived from animal cell (e.g., CHO cell) line expressing the protein A of the present invention (e.g., ELOVL2) at a high level as an enzyme source are added to the solution. After agitation, the mixture is incubated at 37°C for 60 minutes. Trichloroacetic acid is then added to become 4% and 1µL of 185kBq [2- ¹⁴C]-malonyl CoA (Amersham BioScience) is further added thereto. The mixture is agitated. The reaction product is recovered onto Unifilter-96 GF/C (Packard) using Harvexter (Packard) and 20 µL of microscinti-20 (Parkin Elmer) is added thereto. After agitation, the radioactivity(e.g., ¹⁴C) of labeled (liberated) fatty acids is determined with TopCount (Packard). When the activity obtained by adding no test compound is made 100, 50% inhibitory concentration (IC₅₀) is determined. A compound which gives a lower IC₅₀ is selected as the compound inhibiting the activity (e.g., the fatty acid elongation activity) of the protein A (e.g., ELOVL2) of the present invention.

In the screening method described above, for example, in the cases of (ia) and (iia), the cell growth promoting activity is assayed by publicly known methods, e.g., by the colony formation assay or with its modifications, etc., followed by comparison.

Specifically, the areas obtained in (ia) the case in which the cells transfected with an expression vector of the protein A of the present invention are incubated (incubated in, e.g., a medium which selects only the transfected cells) and the formed colony is stained, and in (iia) the case in which a test compound and the cells transfected with an expression vector of the protein A of the present invention are incubated (incubated in, e.g., a medium which selects only the transfected cells) and the formed colony is stained are determined, followed by comparison.

As the cells capable of producing the protein A of the present invention, there are used, for example, the aforesaid host (transformant) transformed with a vector containing the DNA encoding the protein A of the present invention. Preferably, animal cells such as COS7 cells, CHO cells, HEK293 cells, MCF-7 cells, etc. are used as the host. For the screening, the transformant, in which the protein A of the present invention has been expressed in the cells, e.g., by culturing through the procedure described above, is preferably employed. The procedure for incubating the cells capable of expressing the protein A of the present invention is similar to the incubation procedure for the transformant of the present invention described above.

Examples of the test compound include peptides, proteins, non-peptide compounds, synthetic compounds, fermentation products, cell extracts, plant extracts, animal tissue extracts, etc.

For example, when a test compound inhibits the fatty acid elongation activity or the cell growth promoting activity in the case (iia) described above by at least about 20%, preferably at least 30% and more preferably at least about 50%, as compared to the case (ia) above, the test compound can be selected as the compound capable of inhibiting the activity of the protein A of the present invention.

The compound having the activity of inhibiting the activity of the protein A of the present invention is useful as a safe and low toxic pharmaceutical for suppressing the physiological activities of the protein of the present invention.

The compound or its salt obtained using the screening method or screening kit of the present invention is the compound selected from, for example, peptides, proteins, non-peptide compounds, synthetic compounds, fermentation products, cell extracts, plant extracts, animal tissue extracts, plasma, etc. The salts of these compounds used are those given above as the salts of the protein of the present invention.

The present invention further provides a method of screening the compound or its salts inhibiting the activity (e.g., a cell growth promoting activity, etc.) of the protein B of the present invention, which comprises using the protein B of the present invention.

Specifically, there is employed the method of screening the compound or its salts inhibiting the activity, which comprises comparing (ib) the cell growth promoting activity of a cell capable of producing the protein B of the present invention with (iib) the cell growth promoting activity of a mixture of a cell capable of producing the protein B of the present invention and a test compound.

In the screening method described above, for example, in the cases of (ib) and (iib), the cell growth promoting activity is assayed by publicly known methods, e.g., by the colony formation assay or with its modifications, etc., followed by comparison.

Specifically, the areas obtained in (ib) the case in which the cells transfected with an expression vector of the protein B of the present invention are incubated (incubated in, e.g., a medium which selects only the transfected cells) and the formed colony is stained, and in (iib) the case in which a test compound and the cells transfected with an expression vector of the protein B of the present invention are incubated (incubated in, e.g., a medium which selects only the transfected cells) and the formed colony is stained are determined, followed by comparison.

As the cells capable of producing the protein B of the present invention, there are used, for example, the aforesaid host (transformant) transformed with a vector containing the DNA encoding the protein B of the present invention. Preferably, animal cells such as COS7 cells, CHO cells, HEK293 cells, etc. are used as the host. For the screening, the transformant, in which the protein B of the present invention has been expressed in the cell membrane, in the cells, e.g., by culturing through the procedure described above, is preferably employed. The procedure for incubating the cells capable of expressing the protein B of the present invention is similar to the incubation procedure for the transformant of the present invention described above.

Examples of the test compound include peptides, proteins, non-peptide compounds, synthetic compounds, fermentation products, cell extracts, plant extracts, animal tissue extracts, etc.

For example, when a test compound inhibits the cell growth promoting activity in the case (iib) described above by at least about 20%, preferably at least 30% and more preferably at least about 50%, as compared to the case (ib) above, the test compound can be selected to be the compound capable of inhibiting the activity of the protein B of the present invention, or a salt of said compound.

The compound having the activity of inhibiting the activity of the protein B of the present invention is useful as a safe and low toxic pharmaceutical for suppressing the physiological activities of the protein of the present invention.

The compound or its salt obtained using the screening method or screening kit of the present invention is the compound selected from, for example, peptides, proteins, non-peptide compounds, synthetic compounds, fermentation products, cell extracts, plant extracts, animal tissue extracts, plasma, etc. The salts of these compounds used are those given above as the salts of the protein of the present invention.

Furthermore, the gene for the protein of the present invention also shows an increased expression in cancer tissues. Accordingly, the compound or its salts inhibiting the expression of the gene for the protein of the present invention can also be used as a prophylactic/therapeutic agent for cancer, e.g., breast cancer, colon cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor, etc. Preferably, the compound or its salts are used as a prophylactic/therapeutic agent for breast cancer, prostate cancer, etc. Furthermore, the compound or its salts inhibiting the expression of the gene for the protein of the present invention can be used as, e.g., apoptosis promoters.

Therefore, the polynucleotide (e.g., DNA) of the present invention is useful as a reagent for screening the compound or its salts inhibiting the expression of the gene for the protein of the present invention.

For the screening, there is a method of screening which comprises comparing (iii) the case that a cell capable of producing the protein of the present invention is incubated and (iv) the case that a cell capable of producing the protein used in the present invention is incubated in the presence of a test compound.

In the screening method described above, the expression level of the gene described above (specifically, the level of the protein of the present invention or the level of mRNA encoding the said protein) is determined in the cases of (iii) and (iv), followed by comparison.

Examples of the test compound and the cells capable of producing the protein of the present invention are the same as described above.

The level of the protein of the present invention can be determined by publicly known methods, e.g., by measuring the aforesaid protein present in the cell extract, etc., using an antibody capable of recognizing the protein of the present invention, in accordance with methods like western blot analysis, ELISA, etc., or their modifications.

The mRNA level can be determined by publicly known methods, e.g., in accordance with methods such as Northern hybridization using a nucleic acid containing the entire or a part of SEQ ID NO: 2 as a probe, or PCR using a nucleic acid containing the entire or a part of SEQ ID NO: 2 as a primer, or modifications thereof.

For example, when a test compound inhibits the gene expression in the case (iv) described above by at least about 20%, preferably at least 30% and more preferably at least about 50%, as compared to the case (iii) above, the test compound can be selected to be the compound capable of inhibiting the expression of the gene for the protein of the present invention.

The screening kit of the present invention comprises the protein used in the present invention, its partial peptide or salts thereof, or the cell capable of producing the protein used in the present invention, or its partial peptide.

The compound or its salts obtained by using the screening method or screening kit of the present invention is the test compound described above, e.g., a compound selected from peptides, proteins, non-peptide compounds, synthetic compounds, fermentation products, cell extracts, plant extracts, animal tissue extracts, plasma, etc., or its salt, which is a compound or its salt inhibiting the activity (e.g., the fatty acid elongation activity, the cell growth promoting activity, etc.) of the protein of the present invention, a compound or its salt inhibiting the expression of the gene for the protein of the present invention.

The salts of these compounds used are those given above as the salts of the protein of the present invention.

The compound or its salts inhibiting the activity of the protein of the present invention and the compound or its salts inhibiting the expression of the gene for the protein of the present invention are useful as pharmaceuticals with extremely less side effects, such as a prophylactic/therapeutic agent for cancer, e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor, etc. (preferably as prophylactic/therapeutic agents for breast cancer, prostate cancer, etc.), or as apoptosis promoters.

Where the compound or its salt obtained by using the screening method or screening kit of the present invention is used as the prophylactic/therapeutic agent described above, these compounds can be converted into pharmaceutical preparations in a conventional manner.

For example, the composition for oral administration includes solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or excipient conventionally used in the field of pharmaceutical preparations. Examples of the vehicle or excipient for tablets are lactose, starch, sucrose, magnesium stearate, etc.

Examples of the composition for parenteral administration are injectable preparations, suppositories, etc. The injectable preparations may include dosage forms such as intravenous, subcutaneous, intracutaneous and intramuscular injections, drip infusions, etc. These injectable preparations may be prepared by methods publicly known. For example, the injectable preparations may be prepared by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is usually filled in an appropriate ampoule. The suppository used for rectal administration may be prepared by blending the aforesaid antibody or its salt with conventional bases for suppositories.

Advantageously, the pharmaceutical compositions for oral or parenteral use described above are prepared into pharmaceutical preparations with a unit dose suited to fit a dose of the active ingredients. Such unit dose preparations include, for example, tablets, pills, capsules, injections (ampoules), suppositories, etc. The amount of the aforesaid compound contained is generally 5 to 500 mg per dosage unit form; it is preferred that the aforesaid antibody is contained in about 5 to about 100 mg especially in the form of injection, and in 10 to 250 mg for the other forms.

Each composition described above may further contain other active components unless formulation causes any adverse interaction with the compound described above.

Since the pharmaceutical preparations thus obtained are safe and low toxic, they can be administered to human or warm-blooded animal (e.g., mouse, rat, rabbit, sheep, swine, bovine, horse, fowl, cat, dog, monkey, chimpanzee, etc.) orally or parenterally.

The dose of the compound or its salts may vary depending upon its action, target disease, subject to be administered, route of administration, etc. For example, when the compound or its salt inhibiting the activity of the protein of the present invention is orally administered for the purpose of treating, e.g., breast cancer, the compound or its salt is generally administered to an adult (as 60 kg body weight) in a daily dose of about 0.1 to about 100 mg, preferably about 1.0 to about 50 mg and more preferably about 1.0 to about 20 mg. In parenteral administration, a single dose of the said compound or its salt may vary depending upon subject to be administered, target disease, etc. When the compound or its salts inhibiting the activity of the protein of the present invention is administered to an adult (as 60 kg body weight) in the form of an injectable preparation for the purpose of treating, e.g., breast cancer, it is advantageous to administer the compound or its salt at cancerous lesions by way of injection in a daily dose of about 0.01 to about 30 mg, preferably about 0.1 to about 20 mg, and more preferably about 0.1 to about 10 mg. For other animal species, the corresponding dose as converted per 60 kg weight can be administered.

The compounds described above can also be used in combination with the existing anticancer agents, whereby the side effects of anticancer agents that cause harms on normal cell are alleviated. In this case, the period for administration is not limited but these agents may be administered to the subject simultaneously or in time intervals. The dose can be appropriately chosen by taking into account the clinically adopted dose as a standard. A ratio of the above compound to the anticancer agent may be appropriately chosen depending upon subject to be administered, route for administration, target disease, conditions, combination, etc.

The existing anticancer agents include, for example, chemotherapeutic drugs, hormone therapeutic drugs, immunotherapeutic drugs, etc.

Chemotherapeutic drugs include, for example, alkylating drugs (e.g., Cyclophosphamide, Iphosphamide, Nimustine, Ranimustine, Carboquone, etc.), antimetabolic drugs (e.g., Methotrexate, 5-Fluorouracil, Tegafur, Carmofur, UFT, Doxyfluridine, Cytarabine, Enocitabine, Mercaptopurine, Mercaptopurine riboside, Thioguanine, etc.), anticancer antibiotics (e.g., Mytomicin, Adriamycin, Daunorubicin, Epirubicin, Pirarubicin, Idarubicin, Bleomycin, Peplomycin, Actinomycin, etc.), plant-derived antitumor agents (e.g., Vincristine, Inblastine, Vindesine, Etoposide, Camptothecine, Irinotecan, etc.), Cisplatin, Carboplatin, Nedaplatin, Paclitaxel, Docetaxel, Estramustine, and the like.

Hormone therapeutics include, for example, adrenocortical hormones (e.g., Prednisolone, Prednisone, Dexamethasone, Cortisone acetate, etc.), estrogens (e.g., Estradiol, Ethynylestradiol, Fosfestrol, Clorotrianisene, etc.), antiestrogens (e.g., Epithiostanol, Mepitiostane, Tamoxifen, Clomiphene, etc.), luteinizing hormones (e.g., Hydroxyprogesterone caproate, Dydrogesterone, Medroxyprogesterone, Norethysterone, Norethindrone, etc.), LHRH derivatives (e.g., Leuprorelin acetate, etc.) and the like.

Immunotherapeutic drugs include, for example, microorganism- or bacterium-derived components (e.g., muramyl dipeptide derivatives, Picibanil, etc.), polysaccharides having an immunopotentiating activity (e.g., lentinan, schizophyllan, krestin, etc.), genetically engineered cytokines (e.g., interferons, interleukin 2 (IL-2), interleukin 12 (IL-12), tumor necrosis factor (TNF), etc.), colony stimulating agents (e.g., granulocyte colony stimulating factor, erythropoietin, etc.) and the like.

Moreover, drugs whose effects of ameliorating cachexia have been confirmed in animal models or clinically, that is, cyclooxygenase inhibitors (e.g., indomethacin, etc.) [Cancer Research, 49, 5935-5939, 1989], progesterone derivatives (e.g., megestrol acetate, etc.) [Journal of Clinical Oncology, 12, 213-225, 1994], glucocorticoids (e.g., dexamethasone, etc.), metoclopramide type drugs, tetrahydrocannabinol type drugs (the same references are all applied as described above), fat metabolism ameliorating agents (e.g., eicosapentaenoic acid, etc.) [British Journal of Cancer, 68, 314-318, 1993], growth hormones, IGF-1, or antibodies to the cachexia-inducing factor TNF-α, LIF, IL-6 or oncostatin M, can also be used in combination with the compounds described above.

### (2) Quantification for the protein of the present invention, it partial peptide or salts thereof

The antibody to the protein of the present invention (hereinafter sometimes merely referred to as the antibody of the present invention) is capable of specifically recognizing the protein of the present invention, and thus can be used for quantification of the protein of the present invention in a test sample fluid, in particular, for quantification by sandwich immunoassay; etc.

That is, the present invention provides:
(i) a method of quantifying the protein of the present invention in a test sample fluid, which comprises competitively reacting the antibody of the present invention, a test sample fluid and a labeled form of the protein of the present invention, and measuring the ratio of the labeled form of the protein of the present invention bound to said antibody; and,
(ii) a method of quantifying the protein of the present invention in a test sample fluid, which comprises reacting a test sample fluid simultaneously or continuously with the antibody of the present invention immobilized on a carrier and another labeled antibody of the present invention, and then measuring the activity of the labeling agent on the insoluble carrier.

In the quantification method (ii) described above, it is preferred that one antibody is capable of recognizing the N-terminal region of the protein of the present invention, while another antibody is capable of reacting with the C-terminal region of the protein of the present invention.

The monoclonal antibody to the protein of the present invention (hereinafter sometimes referred to as the monoclonal antibody of the present invention) can be used to quantify the protein of the present invention. In addition, the protein can be detected by means of a tissue staining as well. For these purposes, the antibody molecule per se may be used or F(ab')₂, Fab' or Fab fractions of the antibody molecule may also be used.

The method of quantifying the protein of the present invention using the antibody of the present invention is not particularly limited. Any quantification method can be used, so long as the amount of antibody, antigen or antibody-antigen complex corresponding to the amount of antigen (e.g., the amount of the protein) in a test sample fluid can be detected by chemical or physical means and the amount of the antigen can be calculated from a standard curve prepared from standard solutions containing known amounts of the antigen. For such an assay method, for example, nephrometry, the competitive method, the immunometric method, the sandwich method, etc. are suitably used and in terms of sensitivity and specificity, it is particularly preferred to use the sandwich method described hereinafter.

Examples of the labeling agent used in the assay method using the labeling substance are radioisotopes, enzymes, fluorescent substances, luminescent substances, and the like. As the radioisotopes, there are used, e.g., [¹²⁵I], [¹³¹I], [³H], [¹⁴C], etc. The enzymes described above are preferably enzymes, which are stable and have a high specific activity, and include, e.g., β-galactosidase, β-glucosidase, an alkaline phosphatase, a peroxidase, malate dehydrogenase, etc. As the fluorescent substances, there are used, e.g., fluorescamine, fluorescein isothiocyanate, etc. As the luminescent substances described above there are used, e.g., luminol, a luminol derivative, luciferin, lucigenin, etc. Furthermore, the biotin-avidin system may be used as well for binding of an antibody or antigen to a labeling agent.

For immobilization of the antigen or antibody, physical adsorption may be used. Chemical binding techniques conventionally used for insolubilization or immobilization of proteins, enzymes, etc. may also be used. For carriers, there are used, e.g., insoluble polysaccharides such as agarose, dextran, cellulose, etc.; synthetic resin such as polystyrene, polyacrylamide, silicon, etc., and glass or the like.

In the sandwich method, the immobilized monoclonal antibody of the present invention is reacted with a test fluid (primary reaction), then with a labeled form of another monoclonal antibody of the present invention (secondary reaction), and the activity of the label on the immobilizing carrier is measured, whereby the amount of the protein of the present invention in the test fluid can be quantified.
The order of the primary and secondary reactions may be reversed, and the reactions may be performed simultaneously or with an interval. The methods of labeling and immobilization can be performed by the methods described above. In the immunoassay by the sandwich method, the antibody used for immobilized or labeled antibodies is not necessarily one species, but a mixture of two or more species of antibody may be used to increase the measurement sensitivity.

In the methods of assaying the protein of the present invention by the sandwich method of the present invention, antibodies that bind to different sites of the protein of the present invention are preferably used as the monoclonal antibodies of the present invention used for the primary and secondary reactions. That is, in the antibodies used for the primary and secondary reactions are, for example, when the antibody used in the secondary reaction recognizes the C-terminal region of the protein of the present invention, it is preferable to use the antibody recognizing the region other than the C-terminal region for the primary reaction, e.g., the antibody recognizing the N-terminal region.

The monoclonal antibodies of the present invention can be used for the assay systems other than the sandwich method, for example, the competitive method, the immunometric method, nephrometry, etc.

In the competitive method, antigen in a test fluid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying each of these immunological methods to the quantification method of the present invention, any particular conditions or procedures are not required. Quantification system for the protein of the present invention or its salts is constructed by adding the usual technical consideration in the art to the conventional conditions and procedures. For the details of these general technical means, reference can be made to the following reviews and texts.

For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing).

As described above, the protein of the present invention can be quantified with high sensitivity, using the antibody of the present invention.

Furthermore, when an increased level of the protein of the present invention is detected by quantifying the level of the protein of the present invention using the antibody of the present invention, it can be diagnosed that one suffers from cancer, for example, colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor (preferably breast cancer, prostate cancer, etc.) or the like; or it is highly likely to suffer from these disease in the future.

Moreover, the antibody of the present invention can be used to detect the protein of the present invention, which is present in a test sample such as a body fluid, a tissue, etc. The antibody can also be used to prepare an antibody column for purification of the protein of the present invention, detect the protein of the present invention in each fraction upon purification, analyze the behavior of the protein of the present invention in the cells under investigation; etc.

### (3) Gene diagnostic agent

By using the DNA of the present invention, e.g., as a probe, an abnormality (gene abnormality) of the DNA or mRNA encoding the protein of the present invention or its partial peptide in human or warm-blooded animal (e.g., rat, mouse, guinea pig, rabbit, fowl, sheep, swine, bovine, horse, cat, dog, monkey, chimpanzee, etc.) can be detected. Therefore, the DNA of the present invention is useful as a gene diagnostic agent for detecting damages to the DNA or mRNA, its mutation, or decreased expression, increased expression, overexpression, etc. of the DNA or mRNA, and so on.

The gene diagnosis described above using the DNA of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)), etc.

When overexpression is detected by, e.g., Northern hybridization or DNA mutation is detected by the PCR-SSCP assay, it can be diagnosed that it is highly likely to suffer from cancer, for example, colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor, etc. (preferably, breast cancer, prostate cancer, etc.).

### (4) Pharmaceutical comprising the antisense polynucleotide

The antisense polynucleotide of the present invention that binds to the DNA of the present invention complementarily to inhibit expression of the DNA is low toxic and can suppress the functions (e.g., the fatty acid elongation activity, the cell growth promoting activity, etc.) of the protein of the present invention or the DNA of the present invention in vivo. Thus, the antisense polynucleotide can be used as a prophylactic/therapeutic agent for cancer, e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor, etc. Preferably, the antisense polynucleotide is used as a prophylactic/therapeutic agent for breast cancer, prostate cancer, etc. The antisense polynucleotide of the present invention promotes apoptosis of cancer cells and can thus be used as, e.g., an apoptosis promoter.

Where the antisense polynucleotide described above is used as the aforesaid prophylactic/therapeutic agent or as the promoter, it can be prepared into pharmaceutical preparations by publicly known methods, which are provided for administration.

For example, when the antisense polynucleotide described above is used, the antisense polynucleotide alone is administered directly, or the antisense polynucleotide is inserted into an appropriate vector such as retrovirus vector, adenovirus vector, adenovirus-associated virus vector, etc., followed by treating in a conventional manner. The antisense polynucleotide may then be administered orally or parenterally to human or mammal (e.g., rat, rabbit, sheep, swine, bovine, cat, dog, monkey, etc.) in a conventional manner. The antisense polynucleotide may also be administered as it stands, or may be prepared in pharmaceutical preparations together with a physiologically acceptable carrier to assist its uptake, which are then administered by gene gun or through a catheter such as a catheter with a hydrogel. Alternatively, the antisense polynucleotide may be prepared into an aerosol, which is topically administered into the trachea as an inhaler.

Further for the purposes of improving pharmacokinetics, prolonging a half-life and improving intracellular uptake efficiency, the antisense polynucleotide described above is prepared into pharmaceutical preparations (injectable preparations) alone or together with a carrier such as liposome, etc. and the preparations may be administered intravenously, subcutaneously, etc.

A dose of the antisense polynucleotide may vary depending on target disease, subject to be administered, route for administration, etc. For example, where the antisense polynucleotide of the present invention is administered for the purpose of treating breast cancer, the antisense polynucleotide is generally administered to an adult (60 kg body weight) in a daily dose of about 0.1 to 100 mg.

In addition, the antisense polynucleotide may also be used as an oligonucleotide probe for diagnosis to examine the presence of the DNA of the present invention in tissues or cells and states of its expression.

As the antisense polynucleotide described above can, the double-stranded RNA containing a part of RNA encoding the protein of the present invention, ribozyme containing a part of RNA encoding the protein of the present invention, etc. can also prevent expression of the gene of the present invention to suppress the in vivo function of the protein used in the present invention or the DNA used in the present invention and thus can be used as a prophylactic/therapeutic agent for cancer, e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor, etc. (preferably, prophylactic/therapeutic agents for breast cancer, prostate cancer, etc.), apoptosis promoters, etc.

The double-stranded RNA can be designed based on a sequence of the polynucleotide of the present invention and manufactured by modifications of publicly known methods (e.g., Nature, 411, 494, 2001).

The ribozyme can be designed based on a sequence of the polynucleotide of the present invention and manufactured by modifications of publicly known methods (e.g., TRENDS in Molecular Medicine, 7, 221, 2001). For example, the ribozyme can be manufactured by ligating a publicly known ribozyme to a part of the RNA encoding the protein of the present invention. A part of the RNA encoding the protein of the present invention includes a portion proximal to a cleavage site on the RNA of the present invention, which may be cleaved by a publicly known ribozyme (RNA fragment).

Where the double-stranded RNA or ribozyme described above is used as the prophylactic/therapeutic agent described above, the double-stranded RNA or ribozyme is prepared into pharmaceutical preparations as in the antisense polynucleotide, and the preparations can be provided for administration.

### (5) Pharmaceutical comprising the antibody of the present invention

The antibody of the present invention can be used as a prophylactic/therapeutic agent (e.g., vaccine, etc.) for cancer, for example, colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor, etc. Preferably, the antibody is used as a prophylactic/therapeutic agent for breast cancer, prostate cancer, etc. Also, the antibody of the present invention can also be used as, e.g., an apoptosis promoter.

Since the aforesaid prophylactic/therapeutic agent for diseases and promoters comprising the antibody of the present invention are safe and low toxic, they can be administered to human or a warm-blooded animal (e.g., mouse, rat, rabbit, sheep, swine, bovine, horse, fowl, cat, dog, monkey, chimpanzee, etc.) orally or parenterally (e.g., intravascularly, subcutaneously, etc.) either as liquid preparations as they are or as pharmaceutical compositions of adequate dosage form. Preferably, they can be administered in the form of vaccine in a conventional manner.

The antibody of the present invention may be administered in itself or as an appropriate pharmaceutical composition. The pharmaceutical composition used for the administration may contain a pharmacologically acceptable carrier with the aforesaid antibody or its salts, a diluent or excipient. Such a composition is provided in the form of pharmaceutical preparations suitable for oral or parenteral administration.

Examples of the composition for parenteral administration are injectable preparations, suppositories, vaccine, etc. The injectable preparations may include dosage forms such as intravenous, subcutaneous, intracutaneous and intramuscular injections, drip infusions, etc. These injectable preparations may be prepared by methods publicly known. The injectable preparations may be prepared, e.g., by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule. The suppository used for rectal administration may be prepared by blending the aforesaid antibody or its salt with conventional bases for suppositories.

Examples of the composition for oral administration include solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or an excipient conventionally used in the field of pharmaceutical preparations. Examples of the vehicle or excipient for tablets are lactose, starch, sucrose, magnesium stearate, etc.

Advantageously, the pharmaceutical compositions for oral or parenteral use described above are prepared into pharmaceutical preparations in a unit dose suited to fit a dose of the active ingredients. Such unit dose preparations include, for example, tablets, pills, capsules, injections (ampoules), suppositories, etc. The amount of the aforesaid antibody contained is generally about 5 to 500 mg per dosage unit form; especially in the form of injection, it is preferred that the aforesaid antibody is contained in about 5 to 100 mg and in about 10 to 250 mg for the other forms.

The dose of the aforesaid prophylactic/therapeutic agent or regulator comprising the antibody of the present invention may vary depending upon subject to be administered, target disease, conditions, route of administration, etc. For example, when it is used for the purpose of treating, e.g., breast cancer in an adult, it is advantageous to intravenously administer the antibody of the present invention in a single dose of about 0.01 to about 20 mg/kg body weight, preferably about 0.1 to about 10 mg/kg body weight and more preferably about 0.1 to about 5 mg/kg body weight in approximately 1 to 5 times a day, preferably in approximately 1 to 3 times a day. In other parenteral administration and oral administration, the prophylactic/therapeutic agent or regulator can be administered in a dose corresponding to the dose given above. When the condition is especially severe, the dose may be increased according to the condition.

The antibody of the present invention may be administered in itself or as an appropriate pharmaceutical composition. The pharmaceutical composition used for the administration may contain a pharmacologically acceptable carrier with the aforesaid antibody or its salts, a diluent or excipient. Such a composition is provided in the form of pharmaceutical preparations suitable for oral or parenteral administration (e.g., intravascular injection, subcutaneous injection, etc.).

Each composition described above may further contain other active components unless formulation causes any adverse interaction with the antibody described above.

### (6a) Concerning "a prophylactic/therapeutic agent for cancer comprising the compound or its salt having the effect of inhibiting the activity of ELOVL2" of the present invention and "a prophylactic/therapeutic agent for cancer comprising the compound or its salt having the effect of inhibiting the expression of ELOVL2" of the present invention

The "compound having the effect of inhibiting the activity of ELOVL2" may be any compound so long as it has the effect of inhibiting the activity of ELOVL2 and is used as a prophylactic/therapeutic agent for cancer, e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor, etc. (preferably, the prophylactic/therapeutic agent for breast cancer, prostate cancer, etc.), apoptosis promoters, or the like.

The "compound having the effect of inhibiting the expression of ELOVL2" may be any compound so long as it has the effect of inhibiting the expression of ELOVL2 and is used as a prophylactic/therapeutic agent for cancer, e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor, etc. (preferably, the prophylactic/therapeutic agent for breast cancer, prostate cancer, etc.), apoptosis promoters, or the like.

The prophylactic/therapeutic agent and apoptosis promoters are manufactured in the same way as described above.

### (6b) Concerning "a prophylactic/therapeutic agent for cancer comprising the compound or its salt having the effect of inhibiting the activity of Staufen homolog 2" of the present invention and "a prophylactic/therapeutic agent for cancer comprising the compound or its salt having the effect of inhibiting the expression of Staufen homolog 2" of the present invention

The "compound having the effect of inhibiting the activity of Staufen homolog 2" may be any compound so long as it has the effect of inhibiting the activity of Staufen homolog 2 and is used as a prophylactic/therapeutic agent for cancer, e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor, etc. (preferably, the prophylactic/therapeutic agent for breast cancer, prostate cancer, etc.), apoptosis promoters, or the like.

The "compound having the effect of inhibiting the expression of Staufen homolog 2" may be any compound so long as it has the effect of inhibiting the expression of Staufen homolog 2 and is used as a prophylactic/therapeutic agent for cancer, e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor, etc. (preferably, the prophylactic/therapeutic agent for breast cancer, prostate cancer, etc.), apoptosis promoters, or the like.

### (7) DNA transgenic animal

The present invention provides a non-human mammal bearing DNA encoding the protein of the present invention, which is exogenous (hereinafter abbreviated as the exogenous DNA of the present invention) or its variant DNA (sometimes simply referred to as the exogenous variant DNA of the present invention).

That is, the present invention provides:
(1) A non-human mammal bearing the exogenous DNA of the present invention or its variant DNA;
(2) The mammal according to (1), wherein the non-human mammal is a rodent;
(3) The mammal according to (2), wherein the rodent is mouse or rat; and,
(4) A recombinant vector containing the exogenous DNA of the present invention or its variant DNA and capable of expressing in a mammal; etc.

The non-human mammal bearing the exogenous DNA of the present invention or its variant DNA (hereinafter simply referred to as the DNA transgenic animal of the present invention) can be prepared by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method, etc. Also, it is possible to transfect the exogenous DNA of the present invention into a somatic cell, a living organ, a tissue cell, or the like by the DNA transfection methods, and utilize the transformant for cell culture, tissue culture, etc. In addition, these cells may be fused with the above-described germinal cell by a publicly known cell fusion method to prepare the DNA transgenic animal of the present invention.

Examples of the non-human mammal that can be used include bovine, swine, sheep, goat, rabbits, dogs, cats, guinea pigs, hamsters, mice, rats, etc. Above all, preferred are rodents, especially mice (e.g., C57B1/6 strain, DBA2 strain, etc. for a pure line and for a cross line, B6C3F₁ strain, BDF₁ strain B6D2F₁ strain, BALB/c strain, ICR strain, etc.), rats (Wistar, SD, etc.) or the like, since they are relatively short in ontogeny and life cycle from a standpoint of creating model animals for human disease.

"Mammals" in a recombinant vector that can be expressed in the mammals include the aforesaid non-human mammals, human, etc.

The exogenous DNA of the present invention refers to the DNA of the present invention that is once isolated and extracted from mammals, not the DNA of the present invention inherently possessed by the non-human mammals.

The mutant DNA of the present invention includes mutants resulting from variation (e.g., mutation, etc.) in the base sequence of the original DNA of the present invention, specifically DNAs resulting from base addition, deletion, substitution with other bases, etc. and further including abnormal DNA.

The abnormal DNA is intended to mean DNA that expresses the abnormal protein of the present invention and exemplified by the DNA that expresses a protein for suppressing the function of the normal protein of the present invention.

The exogenous DNA of the present invention may be any one of those derived from a mammal of the same species as, or a different species from, the mammal as the target animal. In transfecting the DNA of the present invention into the target animal, it is generally advantageous to use the DNA as a DNA construct in which the DNA is ligated downstream a promoter capable of expressing the DNA in the target animal. For example, in the case of transfecting the human DNA of the present invention, a DNA transgenic mammal that expresses the DNA of the present invention to a high level, can be prepared by microinjecting a DNA construct (e.g., vector, etc.) ligated with the human DNA of the present invention into a fertilized egg of the target non-human mammal downstream various promoters which are capable of expressing the DNA derived from various mammals (e.g., rabbits, dogs, cats, guinea pigs, hamsters, rats, mice, etc.) bearing the DNA of the present invention highly homologous to the human DNA.

As expression vectors for the protein of the present invention, there are Escherichia coli-derived plasmids, Bacillus subtilis-derived plasmids, yeast-derived plasmids, bacteriophages such as λ phage, retroviruses such as Moloney leukemia virus, etc., and animal viruses such as vaccinia virus, baculovirus, etc. Of these vectors, Escherichia coli-derived plasmids, Bacillus subtilis-derived plasmids, or yeast-derived plasmids, etc. are preferably used.

Examples of these promoters for regulating the DNA expression described above include (1) promoters for DNA derived from viruses (e.g., simian virus, cytomegalovirus, Moloney leukemia virus, JC virus, breast cancer virus, poliovirus, etc.), and (2) promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), protein chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human protein elongation factor 1α (EF-1α) promoters, human and fowl β actin promoters, etc., which are capable of high expression in the whole body are preferred.

Preferably, the vectors described above have a sequence that terminates the transcription of the desired messenger RNA in the DNA transgenic animal (generally termed a terminator); for example, a sequence of each DNA derived from viruses and various mammals, and SV40 terminator of the simian virus and the like are preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for the normal protein of the present invention can be obtained using as a starting material the entire genomic DNA or its portion of liver, kidney, thyroid cell or fibroblast origin from human or various mammals (e.g., rabbits, dogs, cats, guinea pigs, hamsters, rats, mice, etc.) or of various commercially available genomic DNA libraries, or using cDNA prepared by a publicly known method from RNA of liver, kidney, thyroid cell or fibroblast origin as a starting material. Also, an exogenous abnormal DNA can produce the translational region through variation of the translational region of normal protein obtained from the cells or tissues described above by point mutagenesis.

The translational region can be prepared by a conventional DNA engineering technique, in which the DNA is ligated downstream the aforesaid promoter and if desired, upstream the translation termination site, as a DNA construct capable of being expressed in the transgenic animal.

The exogenous DNA of the present invention is transfected at the fertilized egg cell stage in a manner such that the DNA is certainly present in all the germinal cells and somatic cells of the target mammal. The fact that the exogenous DNA of the present invention is present in the germinal cells of the animal prepared by DNA transfection means that all offspring of the prepared animal will maintain the exogenous DNA of the present invention in all of the germinal cells and somatic cells thereof. The offspring of the animal that inherits the exogenous DNA of the present invention also have the exogenous DNA of the present invention in all of the germinal cells and somatic cells thereof.

The non-human mammal in which the normal exogenous DNA of the present invention has been transfected can be passaged as the DNA-bearing animal under ordinary rearing environment, by confirming that the exogenous DNA is stably retained by crossing.

By the transfection of the exogenous DNA of the present invention at the fertilized egg cell stage, the DNA is retained to be excess in all of the germinal and somatic cells. The fact that the exogenous DNA of the present invention is excessively present in the germinal cells of the prepared animal after transfection means that the DNA of the present invention is excessively present in all of the germinal cells and somatic cells thereof. The offspring of the animal that inherits the exogenous DNA of the present invention have excessively the DNA of the present invention in all of the germinal cells and somatic cells thereof.

It is possible to obtain homozygotic animals having the transfected DNA in both homologous chromosomes and breed male and female of the animal so that all the progeny have this DNA in excess.

In a non-human mammal bearing the normal DNA of the present invention, the normal DNA of the present invention has expressed at a high level, and may eventually develop hyperfunction in the function of the protein of the present invention by accelerating the function of endogenous normal DNA. Therefore, the animal can be utilized as a pathologic model animal for such a disease. For example, using the normal DNA transgenic animal of the present invention, it is possible to elucidate the mechanism of hyperfunction in the function of the protein of the present invention and the pathological mechanism of the disease associated with the protein of the present invention and to investigate how to treat these diseases.

Furthermore, since a mammal transfected with the exogenous normal DNA of the present invention exhibits an increasing symptom of the protein of the present invention liberated, the animal is usable for screening test of prophylactic/therapeutic agents for diseases associated with the protein of the present invention, for example, the prophylactic/therapeutic agent for cancer such as colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor, etc.

On the other hand, a non-human mammal having the exogenous abnormal DNA of the present invention can be passaged under normal breeding conditions as the DNA-bearing animal by confirming stable retention of the exogenous DNA via crossing. Furthermore, the exogenous DNA of interest can be utilized as a starting material by inserting the DNA into the plasmid described above. The DNA construct with a promoter can be prepared by conventional DNA engineering techniques. The transfection of the abnormal DNA of the present invention at the fertilized egg cell stage is preserved to be present in all of the germinal and somatic cells of the target mammal. The fact that the abnormal DNA of the present invention is present in the germinal cells of the animal after DNA transfection means that all of the offspring of the prepared animal have the abnormal DNA of the present invention in all of the germinal and somatic cells. Such an offspring that passaged the exogenous DNA of the present invention will have the abnormal DNA of the present invention in all of the germinal and somatic cells. A homozygous animal having the introduced DNA on both of homologous chromosomes can be acquired, and by crossing these male and female animals, all the offspring can be bred to retain the DNA.

In a non-human mammal bearing the abnormal DNA of the present invention, the abnormal DNA of the present invention has expressed to a high level, and may eventually develop the function inactive type inadaptability to the protein of the present invention by inhibiting the functions of endogenous normal DNA. Therefore, the animal can be utilized as a pathologic model animal for such a disease. For example, using the abnormal DNA transgenic animal of the present invention, it is possible to elucidate the mechanism of the function inactive type inadaptability to the protein of the present invention and the pathological mechanism of the disease associated with the protein of the present invention and to investigate how to treat the disease.

More specifically, the transgenic animal of the present invention expressing the abnormal DNA of the present invention at a high level is expected to serve as an experimental model to elucidate the mechanism of the functional inhibition (dominant negative effect) of a normal protein by the abnormal protein of the present invention in the function inactive type inadaptability of the protein of the present invention.

Since a mammal bearing the abnormal exogenous DNA of the present invention shows an increased symptom of the protein of the present invention liberated, the animal is also expected to serve for screening test of prophylactic/therapeutic agents for the function inactive type inadaptability of the protein of the present invention, e.g., prophylactic/therapeutic agents for cancer such as colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor, etc.

Other potential applications of two kinds of the DNA transgenic animals of the present invention described above further include:
(1) Use as a cell source for tissue culture;
(2) Elucidation of the relation to a protein that is specifically expressed or activated by the protein of the present invention, by direct analysis of DNA or RNA in tissues of the DNA transgenic animal of the present invention or by analysis of the polypeptide tissues expressed by the DNA;
(3) Research on the function of cells derived from tissues that are usually cultured only with difficulty, using cells in tissues bearing the DNA cultured by a standard tissue culture technique;
(4) Screening a drug that enhances the functions of cells using the cells described in (3) above; and,
(5) Isolation and purification of the variant protein of the present invention and preparation of an antibody thereto.

Furthermore, clinical conditions of a disease associated wit the protein of the present invention, including the function inactive type inadaptability to the protein of the present invention can be determined by using the DNA transgenic animal of the present invention. Also, pathological findings on each organ in a disease model associated with the protein of the present invention can be obtained in more detail, leading to the development of a new method for treatment as well as the research and therapy of any secondary diseases associated with the disease.

It is also possible to obtain a free DNA-transfected cell by withdrawing each organ from the DNA transgenic animal of the present invention, mincing the organ and degrading with a proteinase such as trypsin, etc., followed by establishing the line of culturing or cultured cells. Furthermore, the DNA transgenic animal of the present invention can serve to identify cells capable of producing the protein of the present invention, and to study in association with apoptosis, differentiation or propagation or on the mechanism of signal transduction in these properties to inspect any abnormality therein. Thus, the DNA transgenic animal can provide an effective research material for the protein of the present invention and for investigation of the function and effect thereof.

To develop a drug for the treatment of diseases associated with the protein of the present invention, including the function inactive type inadaptability to the protein of the present invention, using the DNA transgenic animal of the present invention, an effective and rapid method for screening can be provided by using the method for inspection and the method for quantification, etc. described above. It is also possible to investigate and develop a method for DNA therapy for the treatment of diseases associated with the protein of the present invention, using the DNA transgenic animal of the present invention or a vector capable of expressing the exogenous DNA of the present invention.

### (8) Knockout animal

The present invention provides a non-human mammal embryonic stem cell bearing the DNA of the present invention inactivated and a non-human mammal deficient in expressing the DNA of the present invention.

Thus, the present invention provides:
(1) A non-human mammal embryonic stem cell in which the DNA of the present invention is inactivated;
(2) The embryonic stem cell according to (1), wherein the DNA is inactivated by introducing a reporter gene (e.g., β-galactosidase gene derived from *Escherichia coli*);
(3) The embryonic stem cell according to (1), which is resistant to neomycin;
(4) The embryonic stem cell according to (1), wherein the non-human mammal is a rodent;
(5) The embryonic stem cell according to (4), wherein the rodent is mouse;
(6) A non-human mammal deficient in expressing the DNA of the present invention, wherein the DNA is inactivated;
(7) The non-human mammal according to (6), wherein the DNA is inactivated by inserting a reporter gene (e.g., β-galactosidase derived from *Escherichia coli*) therein and the reporter gene is capable of being expressed under control of a promoter for the DNA of the present invention;
(8) The non-human mammal according to (6), which is a rodent;
(9) The non-human mammal according to (8), wherein the rodent is mouse; and,
(10) A method of screening a compound that promotes or inhibits (preferably inhibits) the promoter activity to the DNA of the present invention, which comprises administering a test compound to the mammal of (7) and detecting expression of the reporter gene.

The non-human mammal embryonic stem cell in which the DNA of the present invention is inactivated refers to a non-human mammal embryonic stem cell that suppresses the ability of the non-human mammal to express the DNA by artificially mutating the DNA of the present invention, or the DNA has no substantial ability to express the protein of the present invention (hereinafter sometimes referred to as the knockout DNA of the present invention) by substantially inactivating the activities of the protein of the present invention encoded by the DNA (hereinafter merely referred to as ES cell).

As the non-human mammal, the same examples as described above apply.

Techniques for artificially mutating the DNA of the present invention include deletion of a part or all of the DNA sequence and insertion of or substitution with other DNA, by genetic engineering. By these variations, the knockout DNA of the present invention may be prepared, for example, by shifting the reading frame of a codon or by disrupting the function of a promoter or exon.

Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, isolating the DNA of the present invention that the desired non-human mammal possesses, inserting a DNA fragment having a DNA sequence constructed by inserting a drug resistant gene such as a neomycin resistant gene or a hygromycin resistant gene, or a reporter gene such as lacZ (β-galactosidase gene) or cat (chloramphenicol acetyltransferase gene), etc. into its exon site thereby to disable the functions of exon, or integrating to a chromosome of the target animal by, e.g., homologous recombination, a DNA sequence that terminates gene transcription (e.g., polyA additional signal, etc.) in the intron between exons, thus inhibiting the synthesis of complete messenger RNA and eventually destroying the gene (hereinafter simply referred to as a targeting vector). The thus-obtained ES cells to the southern hybridization analysis with a DNA sequence on or near the DNA of the present invention as a probe, or to PCR analysis with a DNA sequence on the targeting vector and another DNA sequence near the DNA of the present invention which is not included in the targeting vector as primers, to select the knockout ES cell of the present invention.

The parent ES cells to inactivate the DNA of the present invention by homologous recombination, etc. may be of a strain already established as described above, or may originally be established in accordance with a modification of the known method by Evans and Kaufman described above. For example, in the case of mouse ES cells, currently it is common practice to use ES cells of the 129 strain. However, since their immunological background is obscure, the C57BL/6 mouse or the BDF₁ mouse (F₁ hybrid between C57BL/6 and DBA/2), wherein the low ovum availability per C57BL/6 in the C57BL/6 mouse has been improved by crossing with DBA/2, may be preferably used, instead of obtaining a pure line of ES cells with the clear immunological genetic background and for other purposes. The BDF₁ mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes at 3.5 days after fertilization are commonly used. In the present invention, embryos are preferably collected at the 8-cell stage, after culturing until the blastocyte stage, the embryos are used to efficiently obtain a large number of early stage embryos.

Although the ES cells used may be of either sex, male ES cells are generally more convenient for generation of a germ cell line chimera. It is also desirable that sexes are identified as soon as possible to save painstaking culture time.

Methods for sex identification of the ES cell include the method in which a gene in the sex-determining region on the Y-chromosome is amplified by the PCR process and detected. When this method is used, one colony of ES cells (about 50 cells) is sufficient for sex-determination analysis, which karyotype analysis, for example G-banding method, requires about 10⁶ cells; therefore, the first selection of ES cells at the early stage of culture can be based on sex identification, and male cells can be selected early, which saves a significant amount of time at the early stage of culture.

Also, second selection can be achieved by, for example, confirmation of the number of chromosomes by the G-banding method. It is usually desirable that the chromosome number of the obtained ES cells be 100% of the normal number. However, when it is difficult to obtain the cells having the normal number of chromosomes due to physical operations, etc. in the cell establishment, it is desirable that the ES cell is again cloned to a normal cell (e.g., in a mouse cell having the number of chromosomes being 2n = 40) after knockout of the gene of the ES cells.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably 5% carbon dioxide and 95% air, or 5% oxygen, 5% carbon dioxide and 90% air) in the presence of LIF (1 to 10000 U/ml) on appropriate feeder cells such as STO fibroblasts, treated with a trypsin/EDTA solution (normally 0.001 to 0.5% trypsin/0.1 to about 5 mM EDTA, preferably about 0.1% trypsin/1 mM EDTA) at the time of passage to obtain separate single cells, which are then plated on freshly prepared feeder cells. This passage is normally conducted every 1 to 3 days; it is desirable that cells be observed at the passage and cells found to be morphologically abnormal in culture, if any, be abandoned.

Where ES cells are allowed to reach a high density in mono-layers or to form cell aggregates in suspension under appropriate conditions, it is possible to differentiate the ES cells to various cell types, for example, pariental and visceral muscles, cardiac muscle or the like [M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology Experimental Morphology, 87, 27, 1985]. The cells deficient in expression of the DNA of the present invention, which are obtained from the differentiated ES cells of the present invention, are useful for studying the function of the protein of the present invention cytologically.

The non-human mammal deficient in expression of the DNA of the present invention can be identified from a normal animal by measuring the mRNA level in the subject animal by a publicly known method, and indirectly comparing the degrees of expression.

As the non-human mammal, the same examples given above apply.

With respect to the non-human mammal deficient in expression of the DNA of the present invention, the DNA of the present invention can be knockout by transfecting a targeting vector, prepared as described above, to mouse embryonic stem cells or mouse oocytes, and conducting homologous recombination in which a targeting vector DNA sequence, wherein the DNA of the present invention is inactivated by the transfection, is replaced with the DNA of the present invention on a chromosome of a mouse embryonic stem cell or mouse embryo.

The knockout cells with the disrupted DNA of the present invention can be identified by the southern hybridization analysis using as a probe a DNA fragment on or near the DNA of the present invention, or by the PCR analysis using as primers a DNA sequence on the targeting vector and another DNA sequence at the proximal region of other than the DNA of the present invention derived from mouse used in the targeting vector. When non-human mammal stem cells are used, a cell line wherein the DNA of the present invention is inactivated by homologous recombination is cloned; the resulting clones are injected to, e.g., a non-human mammalian embryo or blastocyst, at an appropriate stage such as the 8-cell stage. The resulting chimeric embryos are transplanted to the uterus of the pseudopregnant non-human mammal. The resulting animal is a chimeric animal constructed with both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a series of offspring obtained by crossing between such a chimeric animal and a normal animal, e.g., by coat color identification, etc. The individuals thus obtained are normally deficient in heterozygous expression of the protein of the present invention. The individuals deficient in homozygous expression of the protein of the present invention can be obtained from offspring of the intercross between those deficient in heterozygous expression of the protein of the present invention.

When an oocyte is used, a DNA solution may be injected, e.g., into the prenucleus by microinjection thereby to obtain a transgenic non-human mammal having a targeting vector introduced in its chromosome. From such transgenic non-human mammals, those having a mutation at the locus of the DNA of the present invention can be obtained by selection based on homologous recombination.

As described above, the individuals in which the DNA of the present invention is knockout permit passage rearing under ordinary rearing conditions, after the individuals obtained by their crossing have proven to have been knockout.

Furthermore, the genital system may be obtained and retained by conventional methods. That is, by crossing male and female animals each having the inactivated DNA, homozygote animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammal embryonic stem cell, in which the DNA of the present invention is inactivated, is very useful for preparing a non-human mammal deficient in expression of the DNA of the present invention.

Since the non-human mammal, in which the DNA of the present invention is inactivated, lacks various biological activities derived from the protein of the present invention, such an animal can be a disease model suspected of inactivated biological activities of the protein of the present invention and thus, offers an effective study to investigate the causes for and therapy for these diseases.

### (8a) Method of screening the compound having a prophylactic/therapeutic effect on diseases caused by deficiency, damages, etc. of the DNA of the present invention

The non-human mammal deficient in expression of the DNA of the present invention can be employed for screening the compound having a prophylactic/therapeutic effect on diseases caused by deficiency, damages, etc. of the DNA of the present invention.

That is, the present invention provides a method of screening the compound having a prophylactic/therapeutic effect on diseases, e.g., cancer, caused by deficiency, damages, etc. of the DNA of the present invention, which comprises administering a test compound to a non-human mammal deficient in expression of the DNA of the present invention and, observing and measuring a change occurred in the animal.

As the non-human mammal deficient in expression of the DNA of the present invention, which can be employed for the screening method, the same examples as described above apply.

Examples of the test compound include peptides, proteins, non-peptide compounds, synthetic compounds, fermentation products, cell extracts, plant extracts, animal tissue extracts, blood plasma, etc. These compounds may be novel compounds or publicly known compounds.

Specifically, the non-human mammal deficient in expression of the DNA of the present invention is treated with a test compound, comparison is made with an intact animal for control and a change in each organ, tissue, disease conditions, etc. of the animal is used as an indicator to assess the prophylactic/therapeutic effects of the test compound.

For treating an animal to be tested with a test compound, for example, oral administration, intravenous injection, etc. are applied, and the treatment can be appropriately selected depending on conditions of the test animal, properties of the test compound, etc. Furthermore, a dose of the test compound to be administered can be appropriately chosen depending on the administration route, nature of the test compound, etc.

For screening of the compound having a prophylactic/therapeutic effect on cancer, e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor, etc., a test compound is administered to the non-human mammal deficient in expression of the DNA of the present invention. Differences in incidence of cancer or differences in degree of healing from the group administered with no test compound are observed in the tissues described above with passage of time.

In the screening method, when a test compound is administered to a test animal and the disease conditions of the test animal are improved by at least about 10%, preferably at least about 30% and more preferably at least about 50%, the test compound can be selected as the compound having the prophylactic/therapeutic effect on the diseases described above.

The compound obtained using the above screening method is a compound selected from the test compounds described above and exhibits a prophylactic/therapeutic effect on diseases caused by deficiencies, damages, etc. of the protein of the present invention. Therefore, the compound can be employed as a safe and low toxic drug for the prevention/treatment of the diseases. Furthermore, compounds derived from the compound obtained by the screening described above may also be used as well.

The compound obtained by the screening method above may form salts, and may be used in the form of salts with physiologically acceptable acids (e.g., inorganic acids, organic acids, etc.) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

A pharmaceutical comprising the compound obtained by the above screening method or salts thereof can be manufactured in a manner similar to the method for preparing the pharmaceutical comprising the protein of the present invention described hereinabove.

Since the pharmaceutical preparation thus obtained is safe and low toxic, it can be administered to human or mammal (e.g., rat, mouse, guinea pig, rabbit, sheep, swine, bovine, horse, cat, dog, monkey, etc.).

The dose of the compound or its salt may vary depending upon target disease, subject to be administered, route of administration, etc. For example, when the compound is orally administered, the compound is administered to the adult patient with breast cancer (as 60 kg body weight) generally in a dose of about 0.1 to 100 mg, preferably about 1.0 to 50 mg and more preferably about 1.0 to 20 mg. In parenteral administration, a single dose of the compound may vary depending upon subject to be administered, target disease, etc. When the compound is administered to the adult patient with breast cancer (as 60 kg body weight) in the form of an injectable preparation, it is advantageous to administer the compound in a single dose of about 0.01 to about 30 mg, preferably about 0.1 to about 20 mg and more preferably about 0.1 to about 10 mg a day. For other animal species, the corresponding dose as converted per 60 kg weight can be administered.

### (8b) Method of screening a compound that promotes or inhibits the activity of a promoter to the DNA of the present invention

The present invention provides a method of screening a compound or its salts that promote or inhibit the activity of a promoter to the DNA of the present invention, which comprises administering a test compound to a non-human mammal deficient in expression of the DNA of the present invention and detecting the expression of a reporter gene.

In the screening method described above, an animal in which the DNA of the present invention is inactivated by introducing a reporter gene and the reporter gene is expressed under control of a promoter to the DNA of the present invention is used as the non-human mammal deficient in expression of the DNA of the present invention, which is selected from the aforesaid non-human mammals deficient in expression of the DNA of the present invention.

The same examples of the test compound apply to specific compounds described above.

As the reporter gene, the same specific examples apply to this screening method. Preferably, there are used β-galactosidase (lacZ), soluble alkaline phosphatase gene, luciferase gene and the like.

Since the reporter gene is present under control of a promoter to the DNA of the present invention in the non-human mammal deficient in expression of the DNA of the present invention wherein the DNA of the present invention is substituted with the reporter gene, the activity of the promoter can be detected by tracing the expression of a substance encoded by the reporter gene.

When a part of the DNA region encoding the protein of the present invention is substituted with, e.g., β-galactosidase gene (lacZ) derived from *Escherichia coli,* β-galactosidase is expressed in a tissue where the protein of the present invention should originally be expressed, instead of the protein of the present invention. Thus, the state of expression of the protein of the present invention can be readily observed in vivo of an animal by staining with a reagent, e.g., 5-bromo-4-chloro-3-indolyl-β-galactopyranoside (X-gal) which is substrate for β-galactosidase. Specifically, a mouse deficient in the protein of the present invention, or its tissue section is fixed with glutaraldehyde, etc. After washing with phosphate buffered saline (PBS), the system is reacted with a staining solution containing X-gal at room temperature or about 37°C for approximately 30 minutes to an hour. After the β-galactosidase reaction is terminated by washing the tissue preparation with 1 mM EDTA/PBS solution, the color formed is observed. Alternatively, mRNA encoding lacZ may be detected in a conventional manner.

The compound or salts thereof obtained using the screening method described above are compounds that are selected from the test compounds described above and that promote or inhibit the promoter activity to the DNA of the present invention.

The compound obtained by the screening method above may form salts, and may be used in the form of salts with physiologically acceptable acids (e.g., inorganic acids, etc.) or bases (e.g., organic acids, etc.) or the like, especially in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The compound or its salts inhibiting the promoter activity to the DNA of the present invention can inhibit expression of the protein of the present invention to inhibit the functions of the protein. Thus, the compound or its salt is useful as prophylactic/therapeutic agents for cancer, e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor, etc.

In addition, compounds derived from the compound obtained by the screening described above may also be used as well.

A pharmaceutical comprising the compound obtained by the above screening method or salts thereof can be manufactured in a manner similar to the method for preparing the pharmaceutical comprising the protein of the present invention described above.

Since the pharmaceutical preparation thus obtained is safe and low toxic, it can be administered to human or mammal (e.g., rat, mouse, guinea pig, rabbit, sheep, swine, bovine, horse, cat, dog, monkey, etc.).

A dose of the compound or salts thereof may vary depending on target disease, subject to be administered, route for administration, etc.; when the compound that inhibits the promoter activity to the DNA of the present invention is orally administered, the compound is administered to the adult patient with breast cancer (as 60 kg body weight) normally in a daily dose of about 0.1 to 100 mg, preferably about 1.0 to 50 mg and more preferably about 1.0 to 20 mg. In parenteral administration, a single dose of the compound varies depending on subject to be administered, target disease, etc. but when the compound of inhibiting the promoter activity to the DNA of the present invention is administered to the adult patient with breast cancer (as 60 kg body weight) in the form of injectable preparation, it is advantageous to administer the compound intravenously to the patient in a daily dose of about 0.01 to about 30 mg, preferably about 0.1 to about 20 mg and more preferably about 0.1 to about 10 mg. For other animal species, the corresponding dose as converted per 60 kg weight can be administered.

As stated above, the non-human mammal deficient in expression of the DNA of the present invention is extremely useful for screening the compound or its salt that promotes or inhibits the promoter activity to the DNA of the present invention and, can greatly contribute to elucidation of causes for various diseases suspected of deficiency in expression of the DNA of the present invention and for the development of prophylactic/therapeutic agents for these diseases.

In addition, a so-called transgenic animal (gene transferred animal) can be prepared by using a DNA containing the promoter region of the protein of the present invention, ligating genes encoding various proteins at the downstream and injecting the same into oocyte of an animal. It is thus possible to synthesize the protein therein specifically and study its activity in vivo. When an appropriate reporter gene is ligated to the promoter site described above and a cell line that expresses the gene is established, the resulting system can be utilized as the search system for a low molecular compound having the action of specifically promoting or inhibiting the in vivo productivity of the protein itself of the present invention.

In the specification and drawings, the codes of bases, amino acids, etc. are denoted in accordance with the IUPAC-IUB Commission on Biochemical Nomenclature or by the common codes in the art, examples of which are shown below. For amino acids that may have the optical isomer, L form is presented unless otherwise indicated.
- DNA :: deoxyribonucleic acid
- cDNA :: complementary deoxyribonucleic acid
- A: : adenine
- T: : thymine
- G: : guanine
- C: : cytosine
- RNA: :ribonucleic acid
- mRNA :: messenger ribonucleic acid
- dATP :: deoxyadenosine triphosphate
- dTTP :: deoxythymidine triphosphate
- dGTP :: deoxyguanosine triphosphate
- dCTP :: deoxycytidine triphosphate
- ATP: : adenosine triphosphate
- EDTA :: ethylenediaminetetraacetic acid
- SDS: : sodium dodecyl sulfate
- Gly: : glycine
- Ala: : alanine
- Val: : valine
- Leu: :leucine
- Ile: : isoleucine
- Ser: : serine
- Thr: : threonine
- Cys: : cysteine
- Met: : methionine
- Glu: : glutamic acid
- Asp: : aspartic acid
- Lys: : lysine
- Arg: : arginine
- His: : histidine
- Phe: : phenylalanine
- Tyr: : tyrosine
- Trp: : tryptophan
- Pro: : proline
- Asn: : asparagine
- Gln: glutamine
- pGlu: : pyroglutamic acid
- Sec: : selenocysteine

Substituents, protecting groups and reagents generally used in this specification are presented as the codes below.
- Me: : methyl group
- Et: : ethyl group
- Bu: : butyl group
- Ph: : phenyl group
- TC: : thiazolidine-4(R)-carboxamido group
- Tos: : p-toluenesulfonyl
- CHO :: formyl
- Bzl: : benzyl
- Cl₂-Bzl :: 2,6-dichlorobenzyl
- Bom :: benzyloxymethyl
- Z: : benzyloxycarbonyl
- Cl-Z: : 2-chlorobenzyloxycarbonyl
- Br-Z: : 2-bromobenzyl oxycarbonyl
- Boc: : t-butoxycarbonyl
- DNP :: dinitrophenol
- Trt: : trityl
- Bum :: t-butoxymethyl
- Fmoc :: N-9-fluorenyl methoxycarbonyl
- HOBt: :1-hydroxybenztriazole
- HOOBt :: 3,4-dihydro-3-hydroxy-4-oxo-1,2,3-benzotriazine
- HONB: : 1-hydroxy-5-norbornene-2,3-dicarboxyimide
- DCC :: N,N'-dicyclohexylcarbodiimide

The sequence identification numbers in the sequence listing of the specification indicates the following sequence.

### [SEQ ID NO: 1]

This shows the amino acid sequence of ELOVL2.

### [SEQ ID NO: 2]

This shows the base sequence of DNA encoding ELOVL2 having the amino acid sequence represented by SEQ ID NO: 1.

### [SEQ ID NO: 3]

This shows the base sequence of DNA containing the full-length gene encoding ELOVL2.

### [SEQ ID NO: 4]

This shows the base sequence of the primer used in EXAMPLE 1.

### [SEQ ID NO: 5]

This shows the base sequence of the primer used in EXAMPLE 1.

### [SEQ ID NO: 6]

This shows the base sequence of the primer used in EXAMPLE 2.

### [SEQ ID NO: 7]

This shows the base sequence of the primer used in EXAMPLE 2.

### [SEQ ID NO: 8]

This shows the base sequence of the primer used in EXAMPLE 3.

### [SEQ ID NO: 9]

This shows the base sequence of the primer used in EXAMPLE 3.

### [SEQ ID NO: 10]

This shows the base sequence of the antisense oligonucleotide used in EXAMPLE 4.

### [SEQ ID NO: 11]

This shows the base sequence of the antisense oligonucleotide for control used in EXAMPLE 4.

### [SEQ ID NO: 12]

This shows the base sequence of the primer used in EXAMPLES 4 and 5.

### [SEQ ID NO: 13]

This shows the base sequence of the primer used in EXAMPLES 4 and 5.

### [SEQ ID NO: 14]

This shows the base sequence of the primer used in EXAMPLE 7.

### [SEQ ID NO: 15]

This shows the base sequence of the primer used in EXAMPLE 7.

### [SEQ ID NO: 16]

This shows the amino acid sequence of STAU2.

### [SEQ ID NO: 17]

This shows the base sequence of DNA encoding STAU2 having the amino acid sequence represented by SEQ ID NO: 16.

### [SEQ ID NO: 18]

This shows the base sequence of DNA containing the full-length gene encoding STAU2.

### [SEQ ID NO: 19]

This shows the base sequence of the primer used in EXAMPLE 11.

### [SEQ ID NO: 20]

This shows the base sequence of the primer used in EXAMPLE 11.

### [SEQ ID NO: 21]

This shows the base sequence of the primer used in EXAMPLE 12.

### [SEQ ID NO: 22]

This shows the base sequence of the primer used in EXAMPLE 12.

### [SEQ ID NO: 23]

This shows the base sequence of the primer used in EXAMPLE 13.

### [SEQ ID NO: 24]

This shows the base sequence of the primer used in EXAMPLE 13.

### [SEQ ID NO: 25]

This shows the base sequence of the antisense oligonucleotide used in EXAMPLE 13.

### [SEQ ID NO: 26]

This shows the base sequence of the oligonucleotide for control used in

### EXAMPLE 13.

Hereinafter the present invention will be described more specifically by referring to EXAMPLES but is not deemed to be limited thereto.

### EXAMPLES

### EXAMPLE 1

### (1) Construction of variant p53 vector

Forward and reverse primers (SEQ ID NO: 4 and SEQ ID NO: 5), in which the 524 base counting from A in translation initiation codon ATG of wild type p53 was replaced from G to A, was produced. Wild type p53 (WTp53) (Genbank Accession No. AF136270) was transfected into the Bam HI- Eco RI site of pcDNA3.1 vector (Invitrogen) to give pcDNA3.1-WTp53 vector. Using this pcDNA3.1-WTp53 vector as a template, the variant p53 (MTp53) gene was prepared on QuikChange™ Site-Directed Mutagenesis Kit (STRATAGENE) using the two primers. The procedures were carried out in accordance with the protocol attached. The gene was digested with restriction enzymes Bam HI and Eco RI. The digestion product was transfected into pIND vector (attached to Ecdysone-Inducible Mammalian Expression System (Invitrogen)) digested with the same enzymes to give pIND-MTp53 vector. In this variant p53, arginine (R) at the amino acid sequence 175 was substituted by histidine (H). For control, pIND-WTp53 vector in which the wild type p53 gene was transfected into pIND vector was prepared as well. For another control, CAT (chloramphenicol acetyltransferase)-transfected pIND-CAT vector (Invitrogen) was also used.

### (2) Construction of variant p53 gene expression regulation cell

Lung cancer-derived cell line H1299 (ATCC) deficient of p53 was plated in a 6 cm Petri dish at a density of 4 x 10⁵, followed by incubation overnight. Using Fugene 6, the pIND-MTp53 vector, pIND-WTp53 vector and pIND-CAT vector obtained in (1) described above were co-transfected with pVgRXR vector [attached to Ecdysone-Inducible Mammalian Expression System (Invitrogen)]. The H1299-MTp53, H1299-WTp53 and H1299-CAT cell lines transfected with the respective genes were selected by Geneticin (GIBCO BRL) and Zeosin (Invitrogen). It was confirmed by western blotting using anti-p53 antibody (Oncogene) that the stable expression cell lines of these H1299-MTp53 and H1299-WTp53 induced the expression of MTp53 and WTp53, respectively, by adding 5 µM of Muristerone A (Invitrogen) thereto.

### (3) GeneChip analysis

In order to clarify a group of genes with their expression induced by variant p53, total RNAs were prepared from H1299-MTp53, H1299-WTp53 or H1299-CAT 48 hours after addition of 5 µM of Muristerone A. These total RNAs as materials was subjected to gene expression analysis using GeneChip (Human Genome U95A, U95B, U95C, U95D, U95E; Affymetrix Corp.). Using the total RNAs extracted from 23 normal tissues (TABLE 1) as materials, gene expression analysis was made in the same way. The experiment was carried out by following the experimental protocol (Expression analysis technical manual) of Affymetrix Corp.

As a result, the ELOVL2 gene was found in H1299 cells (H1299-Mtp53 (48hr)), which induced the expression of variant p53, as a gene showing increased expression, when compared to H1299 cells which induced the expression of wild type p53 and H1299 cells which induced the expression of CAT [H1299-Wtp53 (48hr) and H1299-CAT (48hr)]. This gene showed the expression level below the detection limit in normal tissues other than testis (TABLE 2).

**TABLE 1**

| Tissue from which RNA was extracted | Distribution source |
|---|---|
| Small intestine | Clontech Laboratories, Inc. |
| Prostate | Clontech Laboratories, Inc. |
| Fat | BioChain Institute, Inc. |
| Skeletal muscle | Clontech Laboratories, Inc. |
| Heart | Clontech Laboratories, Inc. |
| Kidney | Clontech Laboratories, Inc. |
| Adrenal gland | Clontech Laboratories, Inc. |
| Liver | Clontech Laboratories, Inc. |
| Pancreas | Clontech Laboratories, Inc. |
| Spleen | Clontech Laboratories, Inc. |
| Trachea | Clontech Laboratories, Inc. |
| Lung | Clontech Laboratories, Inc. |
| Whole brain | Clontech Laboratories, Inc. |
| Cerebellum | Clontech Laboratories, Inc. |
| Thymus | Clontech Laboratories, Inc. |
| Mammary gland | Clontech Laboratories, Inc. |
| Salivary gland | Clontech Laboratories, Inc. |
| Stomach | Clontech Laboratories, Inc. |
| Rectum | BioChain Institute, Inc. |
| Colon | BioChain Institute, Inc. |
| Uterus | Clontech Laboratories, Inc. |
| Uterine cervix | BioChain Institute, Inc. |
| Testis | Clontech Laboratories, Inc. |

**TABLE 2**

| Tissue | Gene expression level^{a} |
|---|---|
| Small intestine | ND |
| Prostate | ND |
| Fat | ND |
| Skeletal muscle | ND |
| Heart | ND |
| Kidney | ND |
| Adrenal gland | ND |
| Liver | ND |
| Pancreas | ND |
| Spleen | ND |
| Trachea | ND |
| Lung | ND |
| Whole brain | 1.5 |
| Cerebellum | 2.5 |
| Thymus | ND |
| Mammary gland | ND |
| Salivary gland | ND |
| Stomach | ND |
| Rectum | ND |
| Colon | ND |
| Uterus | ND |
| Uterine cervix | ND |
| Testis | 6.6 |
| H1299-Mtp53 (48hr) | 2.6 |
| H1299-Wtp53 (48hr) | 0.9 |
| H1299-CAT (48hr) | 0.8 |
| ND: not detected | |

| | |
|---|---|
| a: The medial value for the expression level of all genes, which expression was detected by GeneChip, was taken as 1 to standardize the gene expression level. | |

### EXAMPLE 2

### Study of the expression level of ELOVL2 gene in human colon cancer, breast cancer and lung cancer tissues

Using as templates cDNAs (BioChain Institute, Inc.) derived from human colon cancer, breast cancer and lung cancer tissues and first-strand cDNAs (Clontech Laboratories, Inc.) derived from healthy volunteers' colon, mammary and lung tissues, the expression level of ELOVL2 gene was examined. These samples were corrected in advance by the expression level of β-actin gene. Using two primers (SEQ ID NO: 6 and SEQ ID NO: 7), PCR was carried out to make comparison of the ELOVL2 gene expression level between the cancer tissues and normal tissues. As a result, patients with markedly enhanced expression of the ELOVL2 gene in the human colon cancer, breast cancer and lung cancer tissues, as compared to the respective normal tissues, were noted.

### EXAMPLE 3

### (1) Construction of animal cell expression vector

Based on the sequence of ELOVL2 gene described in Genbank NM_017770, two primers (SEQ ID NO: 8 and SEQ ID NO: 9) were prepared and PCR was carried out with pyrobest DNA polymerase (Takara Shuzo Co., Ltd.) using as a template cDNA prepared from H1299-MTp53 added with 5 µM of Muristerone A to give the DNA fragment. This gene segment and pCDNA3.1 (+) plasmid (Invitrogen) were digested with restriction enzymes EcoRV and Notl and the resulting fragments were ligated using TaKaRa Ligation Kit Ver.2 (Takara Shuzo Co., Ltd.). The plasmid DNA obtained was transfected into Escherichia coli DH5α by publicly known methods to give the transformants (clones). A plasmid was extracted from the transformants. After the base sequence of the insert was confirmed using a sequencer (Applied Biosystems, Inc.: ABI3100), animal cell expression vector pCDNA3.1 (+)-ELOVL2 was constructed.

### (2) Effect of cell growth by ELOVL2 gene

Human breast cancer cell line MCF7 (ATCC) and human lung cancer cell line H1299 (ATCC) were plated in a 6 cm Petri dish at a density of 1.1 x 10⁵ each, followed by incubation overnight. Using FuGene 6 (Roche), the pCDNA3.1 (+)-ELOVL2 obtained above and pCDNA3.1 (+)-LacZ (Invitrogen) were transfected according to the protocol attached. After overnight incubation, Geneticin (Invitrogen) was added to the medium, followed by incubation for 6 days. Thereafter the cells were again plated in a 6 cm Petri dish, and incubated for further 3 weeks in a medium supplemented with Geneticin. After washing with PBS, the cells were fixed with 10% neutral formalin solution (Wako Pure Chemical Industries, Ltd.) at room temperature for 30 minutes and washed with PBS. The cells were then stained with crystal violet (SIGMA) solution (crystal violet was dissolved in ethanol to become 10% and the solution was diluted with PBS to 100-fold) at room temperature for 2 hours, washed with distilled water and air-dried. The area of the stained cells was determined by ImagPro Application (Media Cybernetics, Inc.).

The results indicate that when the area of the group in which pCDNA3.1 (+)-LacZ was transfected into MCF7 cells was made 100%, the area of the pCDNA3.1(+)-ELOVL2-transfected group was 170% (P<0.01). When the area of the group in which pCDNA3.1(+)-LacZ was transfected into H1299 cells was made 100%, the area of the pCDNA3.1(+)-ELOVL2-transfected group was 130% (P<0.01).

### EXAMPLE 4

### Cell death by antisense oligonucleotide

In order to analyze effects of the ELOVL2 gene on apoptosis, an experiment to transfect the ELOVL2 antisense oligonucleotide was carried out.

First, an antisense (SEQ ID NO: 10) to the base sequence represented by SEQ ID NO: 3 was designed. Thereafter phosphorothioated oligonucleotide was synthesized, purified on HPLC and used in the transfection run (Amersham Pharmacia Biotech). The reverse sequence (SEQ ID NO: 11) of the base sequence represented by SEQ ID NO: 10 was likewise phosphorothioated and purified on HPLC to use as the oligonucleotide for control (Amersham Pharmacia Biotech).

Breast cancer cell line MCF7 (Dainippon Pharmaceutical Co., Ltd.) was used as test cells. The cells of 1.5 x 10⁴ were plated in a 24-well plate (Falcon Co., Ltd.) on the preceding day of oligonucleotide transfection. For the oligonucleotide transfection, OligofectAMINE (Invitrogen Corp.) was used and its protocol was followed. RNA was extracted in 20 hours after the transfection according to the protocol of RNeasy mini kit (Qiagen, Inc.), and cDNA was prepared using TaqMan Reverse Transcription Reagents (Applied Biosystems, Inc.). For the purposes of determining the ELOVL2 gene expression level using two primers (SEQ ID NO: 12 and SEQ ID NO: 13) and the β-actin expression level for correction using TaqMan human β-actin control reagent (Applied Biosystems, Inc.), PCR was carried out in accordance with the manual of ABI7700 (Applied Biosystems, Inc.). The reaction solution used in the reaction to determine the ELOVL2 gene expression level had the composition of a 15 µl reaction volume containing 7.5 µl of SYBR PCR Master Mix (Applied Biosystems, Inc.), 5 µl of the cDNA template prepared above which was diluted to 3-fold with sterile distilled water and 0.5 µM each of the two primers (SEQ ID NO: 12 and SEQ ID NO: 13). PCR was performed with one cycle at 50°C for 2 minutes, one cycle at 95°C for 10 minutes, followed by repeating 40 cycles at 95°C for 15 seconds and 60°C for 1 minute. The expression level of ELOVL2 gene was corrected by the expression level of β-actin and then provided for comparison.

On the other hand, the effect on apoptosis was assessed on day 3 after the antisense oligonucleotide transfection by comparing with the oligonucleotide-transfected sample for control, using Cell death detection ELISA (Roche).

As a result, the expression of ELOVL2 (RNA) in 20 hours after the antisense oligonucleotide transfection decreased to 56%, as compared when the control (reverse) oligonucleotide was transfected (100%). On day 3 after the transfection, apoptosis increased to 210% when compared with control (100%).

By the foregoing results it was established that when the expression of ELOVL2 was suppressed, cancer cells induced apoptosis, leading to cell death.

### EXAMPLE 5

### (1) Acquisition of MCF-7 cell line expressing ELOVL2 at a high level

Human breast cancer cell line MCF-7 (ATCC) was plated in a 6-well plate at 1.1 x 10⁵. After overnight incubation, pcDNA3.1 (+)-ELOVL2 obtained in EXAMPLE 3 was transfected into the cells using FuGene 6 (Roche). After overnight incubation, Geneticin (Invitrogen Corp.) was added to the culture, followed by incubation for 3 days. The cells were then recovered, diluted and again plated in 15 sets of 96-well plate. The medium was exchanged every 3 other days. Fifteen days after, 96 cell lines which formed single colonies were again plated in 2 sets each of 24-well plate, followed by incubation for further 6 days.

The cells were recovered from one out of 2 wells where the cells were plated. Using RNeasy mini kit (Qiagen, Inc.), RNA was extracted in accordance with the protocol attached and cDNA was prepared using TaqMan Reverse Transcription Reagents (Applied Biosystems, Inc.). For the purposes of determining the ELOVL2 gene expression level using two primers (SEQ ID NO: 12 and SEQ ID NO: 13) and the β-actin expression level for correction using TaqMan human β-actin control reagent (Applied Biosystems, Inc.), PCR was carried out in accordance with the manual of ABI7700 (Applied Biosystems, Inc.). The reaction solution used in the reaction to determine the ELOVL2 gene expression level had the composition of a 15 µl reaction volume containing 7.5 µl of SYBR PCR Master Mix (Applied Biosystems, Inc.), 5 µl of the cDNA template prepared above which was diluted to 3-fold with sterile distilled water and 0.5 µM each of the two primers (SEQ ID NO: 12 and SEQ ID NO: 13). PCR was performed with one cycle at 50°C for 2 minutes, one cycle at 95°C for 10 minutes, followed by repeating 40 cycles at 95°C for 15 seconds and 60°C for 1 minute. The expression level of ELOVL2 gene was corrected by the expression level of β-actin and then provided for comparison. As a result, two cell lines showing the expression of ELOVL2 higher by 26 times and 34 times than the MCF-7 cell line were selected and named MCF7-ELOVL2-C2 and MCF7-ELOVL2-C12, respectively.

### (2) Enhanced cell growth ability in MCF-7 cell line expressing ELOVL2 at a high level

MCF7-ELOVL2-C2 and MCF7-ELOVL2-C12 cells prepared in EXAMPLE 5 (1) above and MCF-7 cells were plated in a 96-well plate at 1 x 10², respectively. On 1 and 7 days after plating, the viable cells were counted in accordance with the attached protocol, using Cell Counting Kit-8 (Dojin Chemical Laboratory). As a result, it was established that MCF7-ELOVL2-C2 and MCF7-ELOVL2-C12 cells enhanced the cell growth rate by 3.1 times and 2.3 times, respectively, when compared to MCF-7 cells.

### (3) Enhanced anchorage-independent cell growth ability in MCF-7 cell line expressing ELOVL2 at a high level

D-MEM medium (Invitrogen Corp.) powders were dissolved in sterile water in a concentration higher by 2-fold than normal concentration to prepare 2 x D-MEM medium. An equal volume of 1.2% agarose (BMA, Inc.) solution to the solution above to prepare the agarose/1 x D-MEM medium of 0.6% final concentration. After dispensing 3 ml each of the medium on a 6-well plate, the mixture was allowed to stand at room temperature for 15 minutes to prepare 0.6% agar medium. The MCF7-ELOVL2-C2 and MCF7-ELOVL2-C12 cells prepared in EXAMPLE 5 (1) above and MCF-7 cells were suspended in 2 x D-MEM medium at 1.2 x 10⁴, respectively. An equal volume of 0.7% agarose (BMA, Inc.) solution was added to the medium (final concentration of 0.35% agarose). Onto the 0.6% agar medium prepared above, 3 ml each of the mixture was dispensed and the medium was allowed to stand at room temperature for 15 minutes to prepare 0.35% agar medium. The medium was incubated at 37°C for 3 weeks in a 5%CO₂ incubator. By examining the size and number of colonies formed and color of the medium, the colony-forming ability was assessed.

As a result, it was established that the colony-forming ability was enhanced in MCF7-ELOVL2-C2 and MCF7-ELOVL2-C12, when compared to MCF7 cells.

### EXAMPLE 6

### Enhanced phosphorylation of MAPK in MCF-7 cell line expressing ELOVL2 at a high level

The MCF7-ELOVL2-C2 and MCF7-ELOVL2-C12 cells prepared in EXAMPLE 5 (1) above and MCF-7 cells were plated in a 10 cm Petri dish at 5 x 10⁵, respectively. After incubation for 4 nights, the medium was removed. One tablet of Complete Mini (Roche) as a protease inhibitor per 20 ml and 200 µl each of RIPA buffer (50 mM Tris-HCl, 150 mM NaCl, 1 % Triton-100, 0.1 % SDS, 1 % Deoxycholic acid) containing 1/1000 volume of PHOSPHATASE INHIBITOR COCKTAIL II (SIGMA) as a phosphatase inhibitor were added to the cells. The cells were scraped off and put in an Eppendorf tube of 1.5 ml. While gently rotating the tube at 4°C, incubation was continued for 30 minutes and centrifugation followed at 15000 rpm for 10 minutes to give the supernatant. A part of the supernatant was taken from the supernatant and its protein level was determined in accordance with the protocol attached, using BCA Protein Assay Kit (PIERCE BIOTECHNOLOGY, INC.). Laemmli Sample Buffer (BIO-RAD) was added to the remaining supernatant, followed by incubation at 95°C for 3 minutes. Samples of the same protein level were subjected to SDS-PAGE and western blotting in a conventional manner. After the blotting membrane was blocked in a conventional manner, the primary antibody and the secondary antibody were reacted with anti-mouse anti-phosphorylated MAPK antibody (Santa Cruz) and with peroxidase-conjugated anti-mouse IgG antibody (Jackson ImmunoResearch Laboratories, Inc.), respectively. Detection was performed on ECL Western Blotting Detection System (Amersham, Inc.). After the membrane was stripped in a conventional manner, the primary antibody and the secondary antibody were reacted with anti-rabbit MAPK antibody (Santa Cruz) and with peroxidase-conjugated anti-rabbit IgG antibody (Jackson ImmunoResearch Laboratories, Inc.), respectively. Detection was performed on ECL Western Blotting Detection System (Amersham, Inc.). As a result, it was established that MCF7-ELOVL2-C2 and MCF7-ELOVL2-C12 enhanced the phosphorylation of MAPK, as compared to MCF-7 cells.

### EXAMPLE 7

### (1) Preparation of animal expression vector with FLAG tag

Two primers (SEQ ID NO: 14 and SEQ ID NO: 15) were designed and PCR was performed with ExTaq (Takara Shuzo Co., Ltd.) using pcDNA3.1-ELOVL2 obtained in EXAMPLE 3 as a template. The reaction was carried out with one cycle at 94°C for 1 minute, followed by repeating 31 cycles at 94°C for 10 seconds, 55°C for 30 seconds and 72°C for 1 minute and 30 seconds. The amplification product obtained was digested with Xbal and EcoRI, which was inserted into plasmid vector p3xFLAG-CMV 10 (SIGMA) digested with the same enzymes, using TaKaRa Ligation Kit Ver.2 (Takara Shuzo Co., Ltd.). Thus, plasmid vector p3xFLAG10-ELOVL2 was obtained.

### (2) Fatty acid elongation activity of ELOVL2

Chinese hamster ovary-derived cell line, i.e., CHO cell line (ATCC) was plated in a 10 cm dish at 1 x 10⁶. After overnight incubation, p3xFLAGIO-ELOVL2 obtained in (1) above or p3xFLAG10 vector (SIGMA) was transfected to the cells using FuGene 6 (Roche). After overnight incubation, the cells were recovered and suspended in buffer A (10 mM Tris-HCl, 0.25M sucrose and 1 mM EDTA) containing one tablet of Complete Mini (Roche) as a protease inhibitor per 20 ml. The cells were homogenized with a polytron homogenizer, followed by centrifugation at 600 gravitational acceleration at 4°C for 10 minutes. The supernatant was centrifuged at 10100 gravitational acceleration at 4°C for 20 minutes. The supernatant was further centrifuged at 126000 gravitational acceleration for 30 minutes. The resulting precipitates were suspended in buffer B (50 mM Tris-HCl and 1 mM EDTA, 20% glycerol) containing one tablet of Complete Mini (Roche) per 20 ml. The suspension was used as an enzyme source.

A fatty acid elongation activity reaction solution [50 mM potassium phosphate/sodium hydroxide (pH 6.5), 5 µM of Rotenone (Wako Pure Chemical Industries, Ltd.), 100 µM of CoA (Wako Pure Chemical Industries, Ltd.), 1 mM ATP, 1 mM NADPH, 1 mM magnesium chloride and 1 µl of 185 kBq [2-¹⁴C]-malonyl CoA (Amersham BioScience, Inc.)] containing 100 µM of 5,8,11,14,17-eicosapentaenoic acid as the substrate fatty acid was prepared, followed by incubation at 37°C for 1 minute. Thereafter, the enzyme source described above was added to the solution in an amount of 50 µg calculated as a protein level. After agitation, the mixture was incubated at 37°C for 20 minutes. The total volume of the reaction solution was made 100 µl. To the reaction solution, 100 µl of 5M KOH (Wako Pure Chemical Industries, Ltd.) containing 10% methanol was added. After incubation at 65°C for an hour, 100 µl of 5M HCl was added thereto and further 100 µl of ethanol was added to the mixture, followed by agitation. Finally, 500 µl of hexane was added. The mixture was vigorously suspended and 400 µl of the upper hexane layer was fractionated. The hexane fraction was charged in a standard vial containing 30 ml of scintillator A (Wako Pure Chemical Industries, Ltd.), followed by stirring. The radioactivity of ¹⁴C was counted with a liquid scintillation counter (Aloka Co., Ltd.).

As a result, the group in which the enzyme source prepared from the p3xFLAG10-ELOVL2-transfected cells was used showed a stronger radioactivity by 3.8 times than control (the enzyme source prepared from the p3xFLAG10 vector-transfected cells).

### EXAMPLE 8

### (1) Acquisition of CHO cell line expressing ELOVL2 at a high level

Chinese hamster ovary-derived cell line, i.e., CHO cell line (ATCC) was plated in a 10 cm dish at 1 x 10⁶. After overnight incubation, p3xFLAG10-ELOVL2 vector obtained in EXAMPLE 7 (1) or p3xFLAG10 vector (SIGMA) was transfected into the cells using FuGene 6 (Roche). After overnight incubation, the medium was replaced by a medium containing 500 µg/ml of Geneticin (Invitrogen Corp.). Incubation was further continued for a week to give ELOVL2 stable expression CHO cell line. Further from the cell line, the monoclonal cell line was produced by limiting dilution. The monoclonal cell line was named CHO-ELOVL2 cell line.

### (2) Construction of high-throughput method for assaying ELOVL2 enzyme activity

The CHO-ELOVL2 cells produced in (1) above were suspended in buffer B (50 mM Tris-HCl and 1 mM EDTA, 20% glycerol) containing one tablet of Complete Mini (Roche) as a protease inhibitor per 20 ml. The cells were homogenated by sonication and the homogenate was centrifuged at 600 gravity acceleration at 4°C for 10 minutes to give the supernatant. The cell extract was used as the enzyme source.

A fatty acid elongation activity reaction solution [50 mM potassium phosphate/sodium hydroxide (pH 6.5), 5 µM Rotenone (Wako Pure Chemical Industries, Ltd.), 100 µM CoA (Wako Pure Chemical Industries, Ltd.), 1mM ATP, 1 mM NADPH, 1 mM magnesium chloride and 1 µl of 185 kBq [2-¹⁴C]-malonyl CoA (Amersham BioScience, Inc.)] containing 100 µM of 5,8,11,14,17-eicosapentaenoic acid as the substrate fatty acid was prepared, and a 90 µl aliquot of the reaction solution was placed in a 96-well plate, followed by incubation at 37°C for 1 minute. Thereafter, the enzyme source described above was added to the solution in an amount of 15 µg (10 µl) calculated as a protein level. After agitation, the mixture was incubated at 37°C for 60 minutes. Then trichloroacetic acid was added to become 4%, followed by agitation. The reaction product was recovered onto Unifilter-96 GF/C (Packard) using Harvexter (Packard) and 20 µL of microscinti-20 (Parkin Elmer) was added thereto. After agitation, the ¹⁴C radioactivity was determined with TopCount (Packard).

As a result, the group in which the enzyme source prepared from the p3xFLAG10-ELOVL2-transfected cells was used showed a stronger radioactivity by 5.2 times than control (the enzyme source prepared from the p3xFLAG10 vector-transfected cells). It could be confirmed that the group had the sensitivity equal to or higher than the elongation activity determined in EXAMPLE 7.

### (3) Substrate specificity of ELOVL2 in fatty acid elongation activity

Using the cell extract derived from the CHO cell line highly expressing ELOVL2 obtained in EXAMPLE 8 (1), the fatty acid elongation activity of ELOVL2 was determined by the high-throughput method for assaying ELOVL2 enzyme activity in EXAMPLE 8 (2), in which cis- 5,8,11,14,1 7-eicosapentaenoic acid, cis-7,10,13,16,19-docosapentaenoic acid, cis-5,8,11,14-eicosatetraenoic acid, arachinonyl-CoA, cis-7,10,13,16-docosatetraenoic acid and cis-5,8,11-eicosatrienoic acid were used as the substrates.

From the foregoing, the fatty acids described above were found to be the substrates for ELOVL2.

### EXAMPLE 9

### Screening

Using the cell extract obtained by the procedure of EXAMPLE 8 (2) as an enzyme solution, screening test was performed.

A fatty acid elongation activity reaction solution [50 mM potassium phosphate/sodium hydroxide (pH 6.5), 5 µM Rotenone (Wako Pure Chemical Industries, Ltd.), 100 µM CoA (Wako Pure Chemical Industries, Ltd.), 1 mM ATP, 1 mM NADPH and 1 mM magnesium chloride] containing 100 µM of 5,8,11,14,17-eicosapentaenoic acid as the substrate fatty acid was prepared, and a 90 µl aliquot of the reaction solution was placed in a 96-well plate, followed by incubation at 37°C for 1 minute. Here, 10 µL of the enzyme solution described above and 2 µL of a solution of test compound in DMF were added. Lastly, 1 µl of 185 kBq [2-¹⁴C]-malonyl CoA (Amersham BioScience, Inc.) was admixed, whereby the ELOVL2 enzyme reaction was initiated. Incubation was carried out at 37°C for 60 minutes. Thereafter, trichloroacetic acid was added to become 4%, followed by agitation. The reaction product was recovered onto Unifilter-96 GF/C (Packard) using Harvexter (Packard) and 20 µL of microscinti-20 (Parkin Elmer) was added thereto. After agitation, the ¹⁴C radioactivity was determined with TopCount (Packard).

Where the activity when the DMF solution obtained by adding no test compound is made 100, 50% inhibitory concentration (IC₅₀) can be determined. A compound which gives a lower IC₅₀ is selected as the compound inhibiting the activity of the protein of the invention.

### EXAMPLE 10

### Cell death by antisense oligonucleotide

Using colon cancer cell line SW480 with little expression of ELOVL2, prostate cancer-derived cell line LNCap cells and prostate cancer-derived cell line PC-3 expressing ELOVL2 as test cells, effects of ELOVL2 on apoptosis by the antisense oligonucleotide were studied as in EXAMPLE 4.

The results indicate that cell death by apoptosis effect was noted in PC-3 as well as in MCF7 cells, whereas in SW480 with little expression of ELOVL2 and in LNCap cells, such cell death by apoptosis effect was not noted.

### EXAMPLE 11

### (1) Construction of variant p53 vector

Forward and reverse primers (SEQ ID NO: 19 and SEQ ID NO: 20), in which the 524 base counting from A in translation initiation codon ATG of wild type p53 was replaced from G to A, was produced. Wild type p53 (WTp53) (Genbank Accession No. AF136270) was transfected into the Bam HI- Eco RI site of pcDNA3.1 vector (Invitrogen) to give pcDNA3.1-WTp53 vector. Using this pcDNA3.1-WTp53 vector as a template, the variant p53 (MTp53) gene was prepared on QuikChange™ Site-Directed Mutagenesis Kit (STRATAGENE) using the two primers. The procedures were carried out in accordance with the protocol attached. The gene was digested with restriction enzymes Bam HI and Eco RI. The digestion product was transfected into pIND vector (attached to Ecdysone-Inducible Mammalian Expression System (Invitrogen)) digested with the same enzymes to give pIND-MTp53 vector. In this variant p53, arginine (R) at the amino acid sequence 175 was substituted by histidine (H). For control, pIND-WTp53 vector in which the wild type p53 gene was transfected into pIND vector was prepared as well. For another control, CAT (chloramphenicol acetyltransferase)-transfected pIND-CAT vector (Invitrogen) was also used.

### (2) Construction of variant p53 gene expression regulation cells

Lung cancer-derived cell line H1299 (ATCC) deficient of p53 was plated in a 6 cm Petri dish at 4 x 10⁵, followed by incubation overnight. Using Fugene 6, the pIND-MTp53 vector, pIND-WTp53 vector and pIND-CAT vector obtained in (1) described above were co-transfected with pVgRXR vector [attached to Ecdysone-Inducible Mammalian Expression System (Invitrogen)]. The H1299-MTp53, H1299-WTp53 and H1299-CAT cell lines transfected with the respective genes were selected by Geneticin (GIBCO BRL) and Zeosin (Invitrogen). It was confirmed by western blotting using anti-p53 antibody (Oncogene) that the stable expression cell lines of these H1299-MTp53 and H1299-WTp53 induced the expression of MTp53 and WTp53, respectively, by adding 5 µM of Muristerone A (Invitrogen) thereto.

### (3) GeneChip analysis

In order to clarify a group of genes with their expression induced by variant p53, total RNAs were prepared from H1299-MTp53, H1299-WTp53 or H1299-CAT 48 hours after addition of 5 µM of Muristerone A. These total RNAs as materials was subjected to gene expression analysis using GeneChip (Human Genome U95A, U95B, U95C, U95D, U95E; Affymetrix Corp.). Using the total RNAs extracted from 23 normal tissues (TABLE 3) as materials, gene expression analysis was made in the same way. The experiment was carried out by following the experimental protocol (Expression analysis technical manual) of Affymetrix Corp.

As a result, the STAU2 gene was found in H1299 cells (H1299-Mtp53 (48hr)), which induced the expression of variant p53, as a gene showing increased expression, when compared to H1299 cells which induced the expression of wild type p53 and H1299 cells which induced the expression of CAT [H1299-Wtp53 (48hr) and H 1299-CAT (48hr)]. This gene showed the expression level below the detection limit in normal tissues (TABLE 4).

**TABLE 3**

| Tissue from which RNA was extracted | Distribution source |
|---|---|
| Small intestine | Clontech Laboratories, Inc. |
| Prostate | Clontech Laboratories, Inc. |
| Fat | BioChain Institute, Inc. |
| Skeletal muscle | Clontech Laboratories, Inc. |
| Heart | Clontech Laboratories, Inc. |
| Kidney | Clontech Laboratories, Inc. |
| Adrenal gland | Clontech Laboratories, Inc. |
| Liver | Clontech Laboratories, Inc. |
| Pancreas | Clontech Laboratories, Inc. |
| Spleen | Clontech Laboratories, Inc. |
| Trachea | Clontech Laboratories, Inc. |
| Lung | Clontech Laboratories, Inc. |
| Whole brain | Clontech Laboratories, Inc. |
| Cerebellum | Clontech Laboratories, Inc. |
| Thymus | Clontech Laboratories, Inc. |
| Mammary gland | Clontech Laboratories, Inc. |
| Salivary gland | Clontech Laboratories, Inc. |
| Stomach | Clontech Laboratories, Inc. |
| Rectum | BioChain Institute, Inc. |
| Colon | BioChain Institute, Inc. |
| Uterus | Clontech Laboratories, Inc. |
| Uterine cervix | BioChain Institute, Inc. |
| Testis | Clontech Laboratories, Inc. |

**TABLE 4**

| Tissue | Gene expression level^{a} |
|---|---|
| Small intestine | ND |
| Prostate | ND |
| Fat | ND |
| Skeletal muscle | ND |
| Heart | ND |
| Kidney | ND |
| Adrenal gland | ND |
| Liver | ND |
| Pancreas | ND |
| Spleen | ND |
| Trachea | ND |
| Lung | ND |
| Whole brain | ND |
| Cerebellum | ND |
| Thymus | ND |
| Mammary gland | ND |
| Salivary gland | ND |
| Stomach | ND |
| Rectum | ND |
| Colon | ND |
| Uterus | ND |
| Uterine cervix | ND |
| Testis | ND |
| H1299-Mtp53 (48hr) | 0.9 |
| H1299-Wtp53 (48hr) | ND |
| H1299-CAT (48hr) | ND |
| ND: not detected | |

| | |
|---|---|
| a: The medial value for the expression level of all genes, which expression was detected by GeneChip, was taken as 1 to standardize the gene expression level. | |

### EXAMPLE 12

### Study of the STAU2 gene expression level in a matched pair of human breast cancer

Using matched pair cDNA (Clontech Laboratories, Inc.) derived from the cancer tissue and normal tissues of human patients with breast cancer as templates, study was made on the STAU2 gene expression level. PCR was carried out using two primers (SEQ ID NO: 21 and SEQ ID NO: 22), whereby comparison in expression level was made between the cancer and normal tissues.

As a result, it was confirmed that there were the patients with marked expression of the STAU2 gene in the cancer tissues, when compared to the normal tissues.

### EXAMPLE 13

### (1) Construction of animal cell expression vector

Based on the sequence of STAU2 gene described in Genbank BC008369, two primers (SEQ ID NO: 23 and SEQ ID NO: 24) were prepared and PCR was carried out with pyrobest DNA polymerase (Takara Shuzo Co., Ltd.) using as a template cDNA prepared from H1299-MTp53 added with 5 µM of Muristerone A to give the DNA fragment. This gene segment and pCDNA3.1 (+) plasmid (Invitrogen) were digested with restriction enzymes EcoRV and Notl and the resulting fragments were ligated using TaKaRa Ligation Kit Ver.2 (Takara Shuzo Co., Ltd.). The plasmid DNA obtained was transfected into Escherichia coli DH5α by publicly known methods to give the transformants (clones). A plasmid was extracted from the transformants. After the base sequence of the insert was confirmed using a sequencer (Applied Biosystems, Inc.: ABI3100), animal cell expression vector pCDNA3.1 (+)-STAU2 was constructed.

### (2) Effect of cell growth by STAU2 gene

Human lung cancer cell line H1299 (ATCC) was plated in each well of a 6-well plate at 1.1 x 10⁵ each, followed by incubation for 24 hours. Using FuGene 6 (Roche), the pCDNA3.1 (+)-STAU2 obtained above and pCDNA3.1 (+)-LacZ (Invitrogen) were transfected according to the protocol attached. After incubation for 24 hours, Geneticin (Invitrogen) was added to the medium, followed by incubation for 6 days. Thereafter the cells were again plated in a 6 cm Petri dish, and incubated for further 3 weeks in a medium supplemented with Geneticin. After washing with PBS, the cells were fixed with 10% neutral formalin solution (Wako Pure Chemical Industries, Ltd.) at room temperature for 30 minutes and washed with PBS. The cells were then dissolved with crystal violet (SIGMA) solution (crystal violet was dissolved in ethanol to become 10% and the solution was diluted with PBS to 100-fold) at room temperature for 2 hours, washed with distilled water and air-dried. The area of the stained cells was determined by ImagPro Application (Media Cybernetics, Inc.). The results indicate that when the area of the group in which pCDNA3.1(+)-LacZ was transfected into H1299 cells was made 100%, the area of the pCDNA3.1(+)-STAU2-transfected group was 110% (P<0.01). From the results it was confirmed that the STAU2 gene promoted the cell growth ability.

### EXAMPLE 14

### Cell death by antisense oligonucleotide

In order to analyze effects of the STAU2 gene on apoptosis, STAU2 antisense oligonucleotide transfection experiment was carried out.

First, an antisense (SEQ ID NO: 25) to the base sequence represented by SEQ ID NO: 18 was designed. Thereafter phosphorothioated oligonucleotide was synthesized, purified on HPLC and used in the transfection experiment (Amersham Pharmacia Biotech). The reverse sequence (SEQ ID NO: 26) of the base sequence represented by SEQ ID NO: 25 was similarly phosphorothioated, purified on HPLC and provided for use as the oligonucleotide for control (Amersham Pharmacia Biotech).

Lung cancer cell line A549 (Dainippon Pharmaceutical Co., Ltd.) was used as test cells. The cells of 3.3 x 10³ were plated in a 96-well plate (Falcon Co., Ltd.) on the preceding day of oligonucleotide transfection. For the oligonucleotide transfection, OligofectAMINE (Invitrogen Corp.) was used and its protocol was followed. On day 3 after the antisense oligonucleotide transfection, comparison was made with the oligonucleotide-transfected sample for control, using Cell death detection ELISA (Roche). Apoptosis increased to 167% when compared with control (100%).

By the foregoing results it was established that when the expression of STAU2 was suppressed, cancer cells induced apoptosis, leading to cell death.

### INDUSTRIAL APPLICABILITY

The protein used in the present invention is expressed specifically in cancer cells and is a diagnostic marker for cancer. Therefore, the compounds or salts thereof inhibiting the activity of the protein, or the compounds or salts thereof inhibiting the expression of a gene for the protein can be safely used as prophylactic/therapeutic agents for cancer, e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor. Preferably, these compounds are prophylactic/therapeutic agents for breast cancer, prostate cancer, etc. Furthermore, the compounds or their salts inhibiting the activity of the protein, or the compounds or salts thereof inhibiting the expression of a gene for the protein can also be used safely as, e.g., apoptosis promoters.

In addition, the antisense polynucleotides or antibodies of the present invention can inhibit the expression of the protein used in the present invention and can be safely used as prophylactic/therapeutic agents for cancer, e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor (preferably, as prophylactic/therapeutic agents for breast cancer, prostate cancer, etc.), or as apoptosis promoters.

## Claims

1. A prophylactic/therapeutic agent for cancer, comprising a compound or its salt inhibiting the activity of a protein comprising the same or substantially the same amino acid sequence as the amino acid sequence represented by SEQ ID NO: 1, its partial peptide, or a salt thereof.

2. A prophylactic/therapeutic agent for cancer, comprising a compound or its salt inhibiting the expression of a gene for a protein comprising the same or substantially the same amino acid sequence as the amino acid sequence represented by SEQ ID NO: 1, or its partial peptide.

3. An antisense polynucleotide containing the entire or part of a base sequence complementary or substantially complementary to a base sequence of a polynucleotide encoding a protein comprising the same or substantially the same amino acid sequence as the amino acid sequence represented by SEQ ID NO: 1, or its partial peptide.

4. The antisense polynucleotide according to claim 3, which has the base sequence represented by SEQ ID NO: 10.

5. A pharmaceutical comprising the antisense polynucleotide according to claim 3.

6. The pharmaceutical according to claim 5, which is a prophylactic/therapeutic agent for cancer.

7. A diagnostic agent comprising the antisense polynucleotide according to claim 3.

8. The diagnostic agent according to claim 7, which is a diagnostic agent for cancer.

9. An antibody to a protein comprising the same or substantially the same amino acid sequence as the amino acid sequence represented by SEQ ID NO: 1, its partial peptide, or a salt thereof.

10. A pharmaceutical comprising the antibody according to claim 9.

11. The pharmaceutical according to claim 10, which is a prophylactic/therapeutic agent for cancer.

12. A diagnostic agent comprising the antibody according to claim 9.

13. The diagnostic agent according to claim 12, which is a diagnostic agent for cancer.

14. A diagnostic agent comprising a polynucleotide encoding a protein comprising the same or substantially the same amino acid sequence as the amino acid sequence represented by SEQ ID NO: 1, or its partial peptide.

15. The prophylactic/therapeutic agent for cancer according to claim 1, 2, 6 or 11, wherein the cancer is colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor.

16. The diagnostic agent according to claim 8, 13 or 14, wherein the cancer is colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor.

17. A prophylactic/therapeutic agent for cancer comprising a compound or its salt inhibiting the activity of ELOVL2.

18. A prophylactic/therapeutic agent for cancer comprising a compound or its salt inhibiting the expression of ELOVL2.

19. A method of screening a prophylactic/therapeutic agent for cancer, which comprises using a protein comprising the same or substantially the same amino acid sequence as the amino acid sequence represented by SEQ ID NO: 1, its partial peptide, or a salt thereof.

20. A kit for screening a prophylactic/therapeutic agent for cancer, comprising a protein comprising the same or substantially the same amino acid sequence as the amino acid sequence represented by SEQ ID NO: 1, its partial peptide, or a salt thereof.

21. A prophylactic/therapeutic agent for cancer, which is obtainable by using the screening method according to claim 19 or the screening kit according to claim 20.

22. A method of screening a prophylactic/therapeutic agent for cancer, which comprises using a polynucleotide encoding a protein comprising the same or substantially the same amino acid sequence as the amino acid sequence represented by SEQ ID NO: 1, or its partial peptide.

23. A kit for screening a prophylactic/therapeutic agent for cancer, comprising a polynucleotide encoding a protein comprising the same or substantially the same amino acid sequence as the amino acid sequence represented by SEQ ID NO: 1, or its partial peptide.

24. A prophylactic/therapeutic agent for cancer, which is obtainable by using the screening method according to claim 22 or the screening kit according to claim 23.

25. An apoptosis promoting agent comprising a compound or its salt inhibiting the activity of a protein comprising the same or substantially the same amino acid sequence as the amino acid sequence represented by SEQ ID NO: 1, its partial peptide, or a salt thereof.

26. An apoptosis promoting agent comprising a compound or its salt inhibiting the expression of a gene for a protein comprising the same or substantially the same amino acid sequence as the amino acid sequence represented by SEQ ID NO: 1, or its partial peptide.

27. An apoptosis promoting agent comprising the antisense polynucleotide according to claim 3.

28. An apoptosis promoting agent comprising the antibody according to claim 9.

29. A method of screening an apoptosis promoter, which comprises using a protein comprising the same or substantially the same amino acid sequence as the amino acid sequence represented by SEQ ID NO: 1, its partial peptide, or a salt thereof.

30. A method of screening an apoptosis promoter, which comprises using a polynucleotide encoding a protein comprising the same or substantially the same amino acid sequence as the amino acid sequence represented by SEQ ID NO: 1, or its partial peptide.

31. A method of preventing/treating cancer, which comprises administering to a mammal an effective dose of a compound or its salt inhibiting the activity of a protein comprising the same or substantially the same amino acid sequence as the amino acid sequence represented by SEQ ID NO: 1, its partial peptide, or a salt thereof, or an effective dose of a compound or its salt inhibiting the expression of a gene for the protein.

32. A method of preventing/treating cancer, which comprises inhibiting the activity of a protein comprising the same or substantially the same amino acid sequence as the amino acid sequence represented by SEQ ID NO: 1, its partial peptide, or a salt thereof, or inhibiting the expression of a gene for the protein.

33. Use of a compound or its salt inhibiting the activity of a protein comprising the same or substantially the same amino acid sequence as the amino acid sequence represented by SEQ ID NO: 1, its partial peptide, or a salt thereof, or a compound or its salt inhibiting the expression of a gene for the protein, to manufacture a prophylactic/therapeutic agent for cancer.

34. A prophylactic/therapeutic agent for cancer comprising a compound or its salt inhibiting the activity of a protein comprising the same or substantially the same amino acid sequence as the amino acid sequence represented by SEQ ID NO: 16, its partial peptide, or a salt thereof.

35. A prophylactic/therapeutic agent for cancer comprising a compound or its salt inhibiting the expression of a gene for a protein comprising the same or substantially the same amino acid sequence as the amino acid sequence represented by SEQ ID NO: 16, or its partial peptide.

36. An antisense polynucleotide containing the entire or part of a base sequence complementary or substantially complementary to a base sequence of a polynucleotide encoding a protein comprising the same or substantially the same amino acid sequence as the amino acid sequence represented by SEQ ID NO: 16, or its partial peptide.

37. The antisense polynucleotide according to claim 36, which has the base sequence represented by SEQ ID NO: 25.

38. A pharmaceutical comprising the antisense polynucleotide according to claim 36.

39. The pharmaceutical according to claim 38, which is a prophylactic/therapeutic agent for cancer.

40. A diagnostic agent comprising the antisense polynucleotide according to claim 36.

41. The diagnostic agent according to claim 40, which is a diagnostic agent for cancer.

42. An antibody to a protein comprising the same or substantially the same amino acid sequence as the amino acid sequence represented by SEQ ID NO: 16, its partial peptide, or a salt thereof.

43. A pharmaceutical comprising the antibody according to claim 42.

44. The pharmaceutical according to claim 43, which is a prophylactic/therapeutic agent for cancer.

45. A diagnostic agent comprising the antibody according to claim 42.

46. The diagnostic agent according to claim 45, which is a diagnostic agent for cancer.

47. A diagnostic agent for cancer comprising a polynucleotide encoding a protein comprising the same or substantially the same amino acid sequence as the amino acid sequence represented by SEQ ID NO: 16, or its partial peptide.

48. The prophylactic/therapeutic agent according to claim 34, 35, 39 or 44, wherein the cancer is colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor.

49. The diagnostic agent according to claim 41, 46 or 47, wherein the cancer is colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, ovarian cancer or blood tumor.

50. A prophylactic/therapeutic agent for cancer comprising a compound or its salt having an action of inhibiting the activity of Staufen homolog 2.

51. A prophylactic/therapeutic agent for cancer comprising a compound or its salt having an action of inhibiting the expression of Staufen homolog 2.

52. A method of screening a prophylactic/therapeutic agent for cancer, which comprises using a protein comprising the same or substantially the same amino acid sequence as the amino acid sequence represented by SEQ ID NO: 16, its partial peptide, or a salt thereof.

53. A kit for screening a prophylactic/therapeutic agent for cancer, comprising a protein comprising the same or substantially the same amino acid sequence as the amino acid sequence represented by SEQ ID NO: 16, its partial peptide, or a salt thereof.

54. A prophylactic/therapeutic agent for cancer, which is obtainable by using the screening method according to claim 52 or the screening kit according to claim 53.

55. A method of screening a prophylactic/therapeutic agent for cancer, which comprises using a polynucleotide encoding a protein comprising the same or substantially the same amino acid sequence as the amino acid sequence represented by SEQ ID NO: 16, or its partial peptide.

56. A kit for screening a prophylactic/therapeutic agent for cancer, comprising a polynucleotide encoding a protein comprising the same or substantially the same amino acid sequence as the amino acid sequence represented by SEQ ID NO: 16, or its partial peptide.

57. A prophylactic/therapeutic agent for cancer, which is obtainable by using the screening method according to claim 55 or the screening kit according to claim 56.

58. An apoptosis promoting agent comprising a compound or its salt inhibiting the activity of a protein comprising the same or substantially the same amino acid sequence as the amino acid sequence represented by SEQ ID NO: 16, its partial peptide, or a salt thereof.

59. An apoptosis promoting agent comprising a compound or its salt inhibiting the expression of a gene for a protein comprising the same or substantially the same amino acid sequence as the amino acid sequence represented by SEQ ID NO: 16, or its partial peptide.

60. An apoptosis promoting agent comprising the antisense polynucleotide according to claim 36.

61. An apoptosis promoting agent comprising the antibody according to claim 42.

62. A method of screening an apoptosis promoter, which comprises using a protein comprising the same or substantially the same amino acid sequence as the amino acid sequence represented by SEQ ID NO: 16, its partial peptide, or a salt thereof.

63. A method of screening an apoptosis promoter, which comprises using a polynucleotide encoding a protein comprising the same or substantially the same amino acid sequence as the amino acid sequence represented by SEQ ID NO: 16, or its partial peptide.

64. A method of preventing/treating cancer, which comprises administering to a mammal an effective dose of a compound or its salt inhibiting the activity of a protein comprising the same or substantially the same amino acid sequence as the amino acid sequence represented by SEQ ID NO: 16, its partial peptide, or a salt thereof, or a compound or its salt inhibiting the expression of a gene for the protein.

65. A method of preventing/treating cancer, which comprises inhibiting the activity of a protein comprising the same or substantially the same amino acid sequence as the amino acid sequence represented by SEQ ID NO: 16, its partial peptide, or a salt thereof, or inhibiting the expression of a gene for the protein.

66. Use of a compound or its salt inhibiting the activity of a protein comprising the same or substantially the same amino acid sequence as the amino acid sequence represented by SEQ ID NO: 16, its partial peptide, or a salt thereof, or a compound or its salt inhibiting the expression of a gene for the protein, to manufacture a prophylactic/therapeutic agent for cancer.
